# EUROPEAN PATENT APPLICATION

(11) **EP 4 219 764 A1**
(43) Date of publication of application: **02.08.2023**
(21) Application number: 22305099.8
(22) Date of filing: 28.01.2022
(51) Int. Cl.: C12Q 1/6886

(54) **METHOD FOR DETECTING METABOLIC REPROGRAMING OF CANCER TOWARDS FATTY ACIDS METABOLISM**

(71) Applicant: Université Paris Cité, 75006 Paris (FR); Institut National de la Santé et de la Recherche Médicale, 75013 Paris (FR)
(72) Inventor: BAUD, Véronique, 75013 PARIS (FR)
(74) Representative: A.P.I. Conseil

(57) **Abstract**

The invention relates to an in vitro method for classifying a subject afflicted with a cancer as suffering from a cancer with (at risk of) a metabolic reprograming towards fatty acids oxidation comprising a step of assaying the activation of RelB in a tumor sample form said cancer. The inventor indeed identified the pivotal role of RelB in energy metabolism and more particularly mitochondrial respiration and fatty acid oxidation. Accordingly, the invention also relates to inhibitors of RelB activity or expression, as well as of lipid metabolism for use in the treatment of cancers showing an activated ReIB.

## Description

### FIELD OF THE INVENTION

The present invention relates to the field of medicine. More particularly the invention relates to a method for detecting metabolic reprograming, or the risk thereof, of a cancer towards fatty acid oxidation. The inventor has indeed identified RelB as a key regulator of metabolism in cancer and the associated gene expression signature of cell metabolic reprograming dependent on RelB activation. Said associated gene expression signature comprises 28 genes. Such information provides valuable information for disease management, more particularly in subject suffering from a refractory cancer. More particularly, the invention also relates to inhibitors of lipid synthesis genes or of fatty acid oxidation for use in cancer cell treatment exhibiting RelB activation dependent metabolic reprograming.

### BACKGROUND OF THE INVENTION

Cancer is the second leading cause of death globally and is responsible for an estimated 9.6 million deaths in 2018. Globally, about 1 in 6 deaths is due to cancer (World Health Organization, <https://www.who.int>). In Europe, cancer incidence is continuously growing and increased by around 50 percent from 2.1 million to 3.1 million cases between 1995 and 2018 (Hofmacher et al., 2019).

Accordingly, the economic impact of cancer is significant and is continuously increasing. Direct costs of cancer doubled from €52 billion to €103 billion in Europe between 1995 and 2018 (Hofmacher et al., 2019), to which should be added significant economic burden of productivity losses due to premature deaths.

Metabolism reprograming is a recognized hallmark of cancer (Hanahan et al, 2011) and confers a selective advantage for tumor cell growth, proliferation, and survival by increasing energy production, macromolecular biosynthesis, and maintenance of redox balance. Reprograming can occur in several metabolism pathways including glucose metabolism and lactate production, glutamine, lipid, amino acid, protein metabolism, oxidative metabolism (DeBerardinis and Chandel, 2016). Metabolic modifications can be selected during tumorigenesis or treatments and can cause cellular adaptations (Ward and Thompson, 2012).

Indeed, in tumor tissue, metabolic heterogeneity induces metabolic symbiosis, which is responsible for adaptation to drastic changes in the nutrient microenvironment resulting from chemotherapy. In addition, metabolic heterogeneity is responsible for the failure to induce the same therapeutic effect against cancer cells as a whole. In particular, cancer stem cells exhibit several biological features responsible for resistance to conventional anti-tumor therapies. Consequently, cancer stem cells tend to form minimal residual disease after chemotherapy and exhibit metastatic potential with additional metabolic reprograming. This type of altered metabolic reprograming leads to adaptive/acquired resistance to anti-tumor therapy (Yoshida 2015).

The most-studied and recognized metabolic reprograming as a hallmark of malignant cancer is the Warburg effect by which cancer cells highly depend of aerobic glycolysis compared to mitochondrial oxidative phosphorylation even when oxygen is abundantly available. This reprograming is necessary for energy production (Warburg, 1956) and thus for tumor cell growth, proliferation and invasion, and resistance to cell death (Dang, 2012). Alterations in lipid metabolism and, more particularly, its redirection towards fatty acid (FA) oxidation to produce cell energy have been more recently shown to be important for carcinogenesis. Indeed, fatty acids functions as signaling molecules, storage, sources of energy and components of the cellular membrane are essential for cancer cells (Currie et al., 2013).

Hence there is a need for tools allowing to detect the presence of cancers with a propensity to metabolic reprograming, to prevent resistance to or to adapt the treatment regimen of the disease. Further, there is a need also for new therapeutic solution that would impair the use of alternative source of energy for the cancer cell and thereby provide valuable therapeutic solutions for cancer.

NF-κB transcription factors family plays a crucial role in the inflammatory and immune response, cell proliferation and survival. In mammals, the NF-κB family is composed of five members, RelA (p65), ReIB, cReI (Rel), NF-κB1 (p50 and its precursor p105) and NF-κB2 (p52 and its precursor p100). These proteins form various homo- and heterodimeric complexes, the activity of which is regulated by two main pathways. The first one, known as the canonical NF-κB activation pathway, mainly applies to RelA and/or cRel containing complexes. The second one, the alternative NF-κB activation pathway, leads to the activation of RelB containing dimers.

It has been surprisingly found that RelB is responsible for metabolic reprograming of cancer cells toward fatty acid oxidation and that inhibition of RelB activity, of fatty acid oxidation or of lipid availability leads to an increase of mortality in cancer cells wherein activation of RelB is detected.

### SUMMARY OF THE INVENTION

As stated above, the inventor surprisingly found that the activation status of the RelB NF-κB subunit is linked to cancer cell energy metabolism and metabolic reprograming. More particularly they show that RelB governs energetic metabolism reprograming to fatty acid oxidation and is controlling cell energy homeostasis by enhancing OxPhos energy metabolism. Therefore, detecting activation of RelB in cancer cell provides a new tool for diagnosing or predicting a risk for developing a cancer with a metabolic reprograming towards fatty acids oxidation. Further, Inventor has been able to detect gene expression signature underlying such metabolic reprograming toward fatty acid oxidation. Lipid metabolism, and particularly fatty acid metabolism, therefore constitutes a valuable target for treating cancers wherein RelB activation and/or such a signature is detected. Accordingly, cancer cell showing an activated RelB are found experimentally sensitive to inhibition of lipid metabolism, thereby providing new and valuable therapeutic options for treating cancer and controlling its evolution.

Accordingly, a first object of the invention relates to an *in vitro* method for classifying a subject afflicted with a cancer as suffering from a cancer with a, or at risk of, metabolic reprograming towards fatty acids oxidation comprising a step of assaying the activation of RelB in a cancer cell sample from said cancer, wherein detecting said activation is indicative of, or of the risk of, the metabolic reprograming of said cancer towards fatty acids oxidation.

In an embodiment said *in vitro* method further comprises a step of detecting an alteration in the expression of at least one gene selected from : CXCL8, NPPB, BAG4, CNTF, SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D.

Indeed, these genes are related to lipid metabolism and their expression is found governed by RelB in cancers with an activated ReIB. Also, identifying, in a cancer cell sample, further to the activation of RelB an alteration of at least one of these genes provide a further indication that said cancer is at risk of evolving, is evolving or has evolved towards metabolism reprograming with a risk of high proliferation rate, resistance to treatment, risk of relapse, gain in aggressiveness (ReIB having been shown has protecting cell against death). Such information is of outmost importance for disease management and to adapt and improve the treatment of the subject. Indeed, RelB dependent lipid metabolism reprograming is found associated with a worse survival expectancy.

The role of RelB in lipid metabolism reprograming, more particularly towards fatty acid oxidation, has been evidenced by the inventor both in solid or non-solid (liquid) cancer, thereby showing the central role of RelB in lipid metabolism in cancer. Accordingly, in an embodiment of said method, said cancer is a solid cancer selected from breast, prostate, pancreatic cancers, glioma, glioblastoma, ovarian, endometrial, skin or liver cancer, or a liquid cancer selected from a Hodgkin lymphoma, a non-Hodgkin lymphoma such as diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL) or leukemia. In a more particular embodiment said cancer is hepatic cancer more preferably hepatocellular carcinoma. In another particular embodiment said cancer is a non-Hodgkin lymphoma, more preferably a DLBCL.

Given its central role in metabolism reprograming of cancer cells, and more particularly in diverting lipid metabolism towards the generation of energy through fatty acid oxidation, inhibition of the expression of RelB activation or of its activity provide a valuable therapeutic target in order to impede said metabolism reprograming. Accordingly, a second object of the invention relates to an inhibitor of the expression of RelB and/or of the activity of RelB for use in inhibiting or preventing metabolic reprograming towards fatty acids oxidation in a cancer cell. In an embodiment, cancer cell shows a metabolic reprograming towards fatty acids oxidation. In another particular embodiment said cancer present at least a part of the RelB dependent at least the RelB dependent lipid metabolism signature ; in said embodiment cancer cell shows an alteration in the expression of at least one gene selected from RELB, CXCL8, NPPB, BAG4, CNTF, SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D.

Any mean to lower or inhibit RelB activity either at the transcriptional or post-translational level is of interest. Antisense nucleotides, siRNA or ShRNA are well-known tools to inhibit gene expression in cells. Also antibodies, and more preferably, small fragments thereof, as e.g. nanobodies, capable of inhibiting RelB activity in NF-κB pathway or e.g. RelB DNA binding activity constitute also a valuable alternative. Accordingly, in an embodiment, the inhibitor of the expression of RelB and/or of the activity of RelB for use in inhibiting or preventing metabolic reprograming towards fatty acids oxidation in a cancer cell comprises at least:
- an antisense nucleotide, a siRNA or a shRNA designed against RelB gene, and/or
- an anti-RelB antibody, a fragment or a derivative thereof.

For the same reason as exposed above, in an embodiment of the inhibitor of the expression of RelB and/or of the activity of RelB for use in inhibiting or preventing metabolic reprograming towards fatty acids oxidation in a cancer cell, said cancer is a solid cancer selected from breast cancer, prostate cancer, pancreatic cancer, glioma, glioblastoma, ovarian, endometrial cancer, skin cancer, liver cancer, or a liquid cancer selected from a Hodgkin lymphoma, a non-Hodgkin lymphoma such as a diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL) or leukemia.

A third object of the invention relates to an inhibitor of lipid metabolism for use in treating a cancer showing an activated ReIB. Indeed cells exhibiting an activated RelB are found more sensitive to lipid metabolism inhibition. Such inhibitor is of particular use in preventing evolution of cancer cell towards reprogramed cancer cell for lipid metabolism and more particularly towards fatty acid oxidation. It constitutes also a valuable therapeutic option against already reprogramed cells which are often more aggressive and resistant to current treatment. Accordingly, in an embodiment, the inhibitor of lipid metabolism for use in treating a cancer showing an activated RelB and further show an alteration in the expression of at least one gene selected from CXCL8, NPPB, BAG4, CNTF, SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D. Said alteration being a clue for an ongoing or an established reprograming of lipid metabolism.

Inhibitors that target different pathway or function in lipid metabolism are of use in treating cancer with an activated ReIB. In a particular embodiment. said inhibitor is selected from at least one compound of the group of soraphen-A, TOFA (5-(tetradecyloxy)-2-furoic acid), A-769662, metformin, [(2R,3S,4R,5R)-5-(5-amino-4-carbamoylimidazol-1-yl)-3,4-dihydroxyoxolan-2-yl]methyl dihydrogen phosphate, SB-204990, LY294002, triacscin C , a thiazolidinedione, TCD-717, MN58B, 1,2,3, benzene-tricarboxylate, etomoxir, ranolazine, ST1326, glyburide, perhexiline, cerulenin, C75, TVB-2640, TVB-3166, orlistat, a flavonoid, epigallocatechin-3-gallate, platensimycin, GSK837149A, JZL-184, (6-[4-(2-Bromo-5-methoxy-benzoyl)-piperazin-1-yl]-N-phenylpropyl-nicotinamide), A939572, fatostatin, FGH10019, betulin, and 25-hydroxycholesterol, or any pharmaceutically acceptable salt or prodrugs thereof, or a combination thereof.

In an embodiment, said inhibitor is for use in treating a cancer selected from breast cancer, prostate cancer, pancreatic cancer, glioma, glioblastoma, ovarian, endometrial cancer, skin cancer, liver cancer, a Hodgkin lymphoma, a non-Hodgkin lymphoma such as a diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL) or leukemia.

### LEGEND OF DRAWINGS

**Figure 1****.** Triglyceride content based on lipid droplet area in HCC Huh7 cells overexpressing RelB (ReIB WT, **A**) or in Huh7 cells transfected with a RelB shRNA (shRNA ReIB, **B**). ****p<0.0001, significantly different from control, unpaired Student's t-test.
**Figure 2****. A** Storage of triglycerides in lipid droplets of HCC Huh7 cells overexpressing RelB (ReIB WT) in the presence of rising concentration of fatty acid; OA oleic acid. **B** Global content in triglycerides (TG) in Huh7 cells overexpressing RelB (ReIB WT) cultured in complete medium (left panel) or supplemented with Oleic Acid (OA, 50 50µM). ***p<0.001; ****p<0.0001 significantly different from control, unpaired Student's t-test.
**Figure 3****. A** Metabolomic study of RelB overexpressing HCC Huh7 cells. **B** [C14]Oleic acid metabolism rates in overexpressing HCC Huh7 cells. CoA : Coenzyme A, TG : triglycerides, PI: phospholipids, ECM : extracellular medium, DAG : diacylglycerol, PL : phospholipids and TG : triglycerides, ASMs : acid soluble metabolites. Arrow down : level significantly lower (ReIB WT vs control); arrow up level significantly higher (ReIB WT vs control).

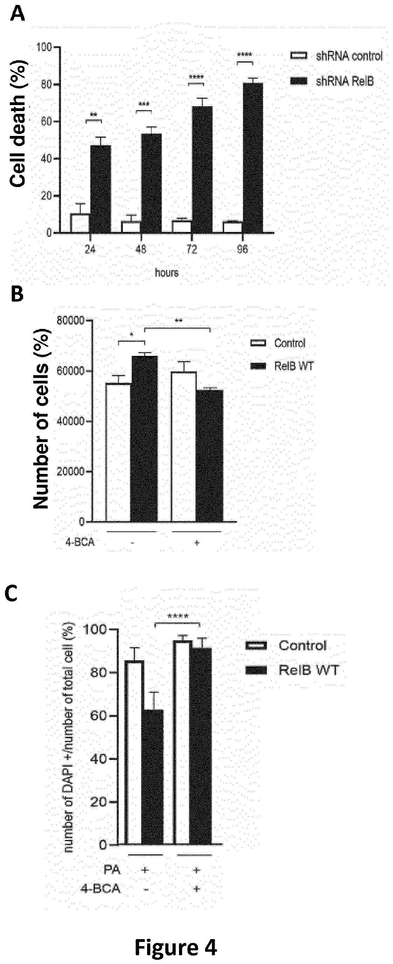

**Figure 4****. A** Cell death in HCC Huh7 cells transduced with a shRNA RelB or control shRNA. **B** Cell growth in RelB overexpressing HCC Huh7 cells at 48h in presence or in the absence of 4-bromocrotonic acid (4-BCA) (10µM, inhibitor of fatty acid oxidation). **C** Cell death quantified at 48h, in 0 % FBS medium + Palmitic Acid (PA, 50µM) for 16h after 5h of FBS starvation, in presence or in absence of 4-BCA and expressed in percentage of positive DAPI cell number. n=3 independent experiments. n=3 experiments, ***p<0.001; ****p<0.0001 significantly different from control, unpaired Student's t-test.
**Figure 5****. A** Kaplan-Meier plot of overall survival of the TCGA cohort (HCC patients, n=364) according to human RelB mRNA expression (RNAseq). P value by Log-rank (Mantel-Cox, <https://kmplot.com>).
**Figure 6****. A** R406 induced apoptosis in MD901 or SUDHL-4 DLBCL cell lines **B.** Levels of Glycolytic or mitochondrial ATP in SUDHL-4 and MD901 cell lines. **p<0.01; ****p<0.0001 significantly different from control, unpaired Student's t-test.
**Figure 7****. A** Cellular ATP content in MD901 cell line upon ReIB-expression knock-down. **B.** Levels of Glycolytic or mitochondrial ATP in MD901 cell line upon ReIB-expression knock-down. **p<0.01 significantly different from control, unpaired Student's t-test.
**Figure 8****. A** Apoptosis induced by inhibitors of electron transport chain metformin (M) and Asparaginase (K) in ReIB-expression knocked-down MD901 cell line **B** Mitochondrial potential in ReIB-expression knocked-down MD901 cell line upon exposure to M or K. Percentage of DAPI positive cells with a low TMRM labelling **p<0.01; ***p<0.001 significantly different from control, unpaired Student's t-test.
**Figure 9****. A** death inhibition of fatty acid oxidation upon exposure to 4-BCA in MD901 cells. **B.** Induction of cell death through exposure to a combination of M/K mix (Metformin (M) (2.5 mM), or Kidrolase^{®} (K) (2 UI/mL)) and 4-BCA (50 µM) in MD901 cells.
**Figure 10****. A** Induction of cell death through inhibition of fatty acid oxidation upon exposure to 4-BCA in OCILY19 cells. **B.** Induction of cell death through exposure to a combination of M/K mix and 4-BCA (30 µM) in OCILY19 cells.
**Figure 11****. A** Induction of cell death through inhibition of Fatty Acid Synthase upon exposure to C-75 in MD901 cells. **B.** Induction of cell death through exposure to a combination of M/K mix (Metformin (M) (2.5 mM), or Kidrolase^{®} (K) (2 UI/mL)) and C-75 (75 µM) in MD901 cells.
**Figure 12****. A** Induction of cell death through inhibition of Fatty Acid Synthase upon exposure to C-75 in OCILY19 cells. **B.** Induction of cell death through exposure to a combination of M/K mix and C-75 (60 µM) in OCILY19 cells.

### DETAILED DESCRIPTION OF THE INVENTION

### Definitions

As intended herein, the term "comprising" has the meaning of "including" or "containing", which means that when an object "comprises" one or several elements, other elements than those mentioned may also be included in the object. In contrast, when an object is said to "consist of" one or several elements, the object cannot include other elements than those mentioned.

**"Cancer"** as used herein refers to any solid or liquid cancer. Solid cancer refers to any carcinoma or sarcoma, for example carcinoma or sarcoma such as lung cancer, breast cancer, prostate cancer, cervical cancer, pancreatic cancer, colon cancer, ovarian cancer; stomach cancer, esophagus cancer, mouth cancer, tongue cancer, gum cancer, skin cancer (e.g., melanoma, basal cell carcinoma, Kaposi's sarcoma, etc.), muscle cancer, heart cancer, liver cancer, bronchial cancer, cartilage cancer, bone cancer, testis cancer, kidney cancer, endometrium cancer, uterus cancer, bladder cancer, spleen cancer, thymus cancer, thyroid cancer, brain cancer, neuron cancer, mesothelioma, gall bladder cancer, ocular cancer (e.g., cancer of the cornea, cancer of uvea, cancer of the choroids, cancer of the macula, vitreous humor cancer, etc.), joint cancer (such as synovium cancer), glioblastoma. More particularly solid cancer refers to breast, prostate, pancreatic cancers, glioma, glioblastoma, ovarian, endometrial, skin and liver cancer. cancer to implement method of the invention are cancers to breast cancers, lung cancers, prostate cancers, colorectal cancers, or bone sarcomas, soft tissue sarcomas. Liver cancer and even more particularly hepatocellular carcinoma (HCC) are particularly preferred solid cancer to implement teaching of the invention. "Liquid cancer" refers to any lymphoma or leukemia. In a preferred embodiment, it refers to an aggressive B-cell lymphoma, either Hodgkin or non-Hodgkin lymphoma, as diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL). In an even more preferred embodiment, liquid cancer is a DLBCL.

By **"metabolic reprograming"** it is meant herein refers to the unusual (in regard with normal cells) changes that are observed in cancer cells that alter their metabolism and lead to support the increased energy request due to continuous growth, rapid proliferation, particular environments of neoplastic cells, migration, invasion, survival and resistance to cancer treatment. An example for such a change is the Warburg effect which refers to the high increase and dependency upon glycolysis, which is observed for certain cancer cells, even in the presence of oxygen. Another example is metabolic reprograming towards fatty acid oxidation.

As used herein **"lipid metabolism"** comprises the various metabolic processes involving or related to lipids either catabolic or anabolic processes. In catabolism, stored lipids are converted into fatty acids which are oxidized via beta oxidation and the citric acid cycle to produce energy (so called FA oxidation), mainly in the form of adenosine triphosphate (ATP). Fatty acids (mainly stored in the form of triglycerides) are therefore the foremost storage form of fuel in most animals. Fatty acids are important precursors to triglycerides, phospholipids, second messengers, hormones and ketone bodies. Also, **"metabolic reprograming towards fatty acid oxidation"** as used herein makes fatty acids the main source of energy for the cells and is associated with (or comprises) the lowering of the storage of lipids, precursors of said fatty acids (e.g. triglycerides). In a very particular embodiment "metabolic reprograming towards fatty acid oxidation" comprises in addition glycolysis lowering and /or OxPhos metabolism enhancement.

By **"activation of ReIB"** it is meant herein the activation of the ReIB-dependent NF-κB pathway, in other words, the transmission of a cellular signal through the RelB signaling cascade of the NF-κB pathway. The NF-κB pathway can act inter alia through the ReIA, RelB or cRel transcription factors. More specifically, the activation of the ReIB-dependent NF-κB pathway of the invention corresponds to the transduction of a cellular signal by the NF-κB pathway through the RelB subunit of the NF-κB complexes and not through the RelA or cRel subunits. Such activation in a cell sample can be easily directly assessed on a cancer cell sample by, for example, Electrophoretic Mobility Shift Assays (EMSA). EMSA combined with supershift allows the detection of DNA binding of RelB containing complexes as described by Jacque et al. (2005). As used herein, "activation of ReIB" can also refer to an increase of the expression level of RelB at the transcriptional, protein level, or both. Alternatively, activation of RelB can be detected by assaying expression of one or more gene from the RelB gene expression signature. Accordingly, a cell showing **"an activated ReIB"** refers herein to a cell wherein DNA binding activity of ReIB, and/or an increase of the expression level of RelB at the transcriptional, protein level, or both is detected.

The terms **"ReIB gene expression signature",** "ReIB gene signature", "ReIB activation signature" or "ReIB signature", refer to the group of genes whom expression is known to be altered upon RelB activation, for example the actual RelB DNA binding activity. In some instance such DNA binding activity can be controlled by the phosphorylation status of the protein.

Also, an "inhibitor of the expression of RelB and/or of the activity of ReIB", as used herein, refers to any compound leading either directly or indirectly at least to a decrease of the signal transduction through the ReIB-dependent NF-κB pathway. This compound can induce i) the lowering or even the extinction of RELB gene expression either at the transcriptional or the translational level, ii) the lowering or the inhibition of RelB protein activity through as an example, post-translational modification (e.g; phosphorylation pattern of the protein on different residues), or inhibition of its DNA binding activity (or of RelB NF-κB complex) through e.g. hindrance of RelB DNA binding site or the induction of conformational changes of ReIB.

As used herein, the term **"subject"** refers to any mammal, e.g. mouse, rat, monkey, dog, human, preferably a human. Preferably, it refers to a human thought to develop or is suspected of suffering from cancer.

"**ReIB**" is a protein which, in humans, is encoded by the *RELB* gene (also known as I-REL, IREL, REL-B, RELB proto-oncogene, IMD53); Human RelB protein sequence is accessible under the Uniprot number Q01201 (NCBI Reference Sequence: NP_006500.2). RelB is conserved through the mammal species and numerous homologs of the human RelB protein of SEQ ID NO:1 exist. Amino acids sequence of human RelB (SEQ ID NO:1) is:

“Met Leu Arg Ser Gly Pro Ala Ser Gly Pro Ser Val Pro Thr Gly Arg Ala Met Pro Ser Arg
Arg Val Ala Arg Pro Pro Ala Ala Pro Glu Leu Gly Ala Leu Gly Ser Pro Asp Leu Ser Ser
Leu Ser Leu Ala Val Ser Arg Ser Thr Asp Glu Leu Glu Ile Ile Asp Glu Tyr Ile Lys Glu Asn
Gly Phe Gly Leu Asp Gly Gly Gln Pro Gly Pro Gly Glu Gly Leu Pro Arg Leu Val Ser Arg
Gly Ala Ala Ser Leu Ser Thr Val Thr Leu Gly Pro Val Ala Pro Pro Ala Thr Pro Pro Pro Trp
Gly Cys Pro Leu Gly Arg Leu Val Ser Pro Ala Pro Gly Pro Gly Pro Gln Pro His Leu Val Ile
Thr Glu Gln Pro Lys Gln Arg Gly Met Arg Phe Arg Tyr Glu Cys Glu Gly Arg Ser Ala Gly
Ser Ile Leu Gly Glu Ser Ser Thr Glu Ala Ser Lys Thr Leu Pro Ala Ile Glu Leu Arg Asp Cys
Gly Gly Leu Arg Glu Val Glu Val Thr Ala Cys Leu Val Trp Lys Asp Trp Pro His Arg Val
His Pro His Ser Leu Val Gly Lys Asp Cys Thr Asp Gly Ile Cys Arg Val Arg Leu Arg Pro

His Val Ser Pro Arg His Ser Phe Asn Asn Leu Gly Ile Gln Cys Val Arg Lys Lys Glu Ile Glu
Ala Ala Ile Glu Arg Lys Ile Gln Leu Gly Ile Asp Pro Tyr Asn Ala Gly Ser Leu Lys Asn His
Gln Glu Val Asp Met Asn Val Val Arg Ile Cys Phe Gln Ala Ser Tyr Arg Asp Gln Gln Gly
Gln Met Arg Arg Met Asp Pro Val Leu Ser Glu Pro Val Tyr Asp Lys Lys Ser Thr Asn Thr
Ser Glu Leu Arg Ile Cys Arg Ile Asn Lys Glu Ser Gly Pro Cys Thr Gly Gly Glu Glu Leu Tyr
Leu Leu Cys Asp Lys Val Gln Lys Glu Asp Ile Ser Val Val Phe Ser Arg Ala Ser Trp Glu
Gly Arg Ala Asp Phe Ser Gln Ala Asp Val His Arg Gln Ile Ala Ile Val Phe Lys Thr Pro Pro
Tyr Glu Asp Leu Glu Ile Val Glu Pro Val Thr Val Asn Val Phe Leu Gln Arg Leu Thr Asp
Gly Val Cys Ser Glu Pro Leu Pro Phe Thr Tyr Leu Pro Arg Asp His Asp Ser Tyr Gly Val
Asp Lys Lys Arg Lys Arg Gly Met Pro Asp Val Leu Gly Glu Leu Asn Ser Ser Asp Pro His
Gly Ile Glu Ser Lys Arg Arg Lys Lys Lys Pro Ala Ile Leu Asp His Phe Leu Pro Asn His Gly
Ser Gly Pro Phe Leu Pro Pro Ser Ala Leu Leu Pro Asp Pro Asp Phe Phe Ser Gly Thr Val
Ser Leu Pro Gly Leu Glu Pro Pro Gly Gly Pro Asp Leu Leu Asp Asp Gly Phe Ala Tyr Asp
Pro Thr Ala Pro Thr Leu Phe Thr Met Leu Asp Leu Leu Pro Pro Ala Pro Pro His Ala Ser
Ala Val Val Cys Ser Gly Gly Ala Gly Ala Val Val Gly Glu Thr Pro Gly Pro Glu Pro Leu Thr
Leu Asp Ser Tyr Gln Ala Pro Gly Pro Gly Asp Gly Gly Thr Ala Ser Leu Val Gly Ser Asn
Met Phe Pro Asn His Tyr Arg Glu Ala Ala Phe Gly Gly Gly Leu Leu Ser Pro Gly Pro Glu
Ala Thr”.

A "ReIB homolog" is a protein whose sequence shares at least 80% homology with the RelB protein of SEQ ID NO:1, while retaining the RelB function, e.g. the capacity of binding p50 or with DNA, that can be demonstrated for example by electrophoretic mobility shift assay (Derruder *et al*., 2003). Preferably, the amino acid sequences of the homologs of the RelB protein are identical at more than 80%, preferably 81%, more preferably 82%, more preferably 83%, more preferably 84%, more preferably 85%, preferably 86%, more preferably 87%, more preferably 88%, more preferably 89%, more preferably 90%, more preferably 91%, more preferably 92%, more preferably 93%, more preferably 94%, more preferably 95%, more preferably 96% to the and even more preferably 97% to SEQ ID NO:1. Preferably, amino acid sequence identity is measured by using the global alignment algorithm of Needleman and Wunsch (1970). Such RelB homolog can be, for example, RelB protein from mouse, rat, monkey, dog.

**"Inhibiting or preventing"** a phenomenon means, as used herein, inducing the absence, the lowering the intensity level or impeding the occurrence of said phenomenon which, in another similar situation, had been present or stronger. Accordingly, **"inhibiting or preventing metabolic reprograming towards fatty acid oxidation"** refers to inducing the absence, the lowering the intensity level or impeding the use of fatty acids catabolism as defined above, and/or the lowering of the storage of lipids to the favor of FA catabolism, and/or, optionally, the lowering of glycolysis pathway and/or enhancement of OxPhos metabolism.

Accordingly, the terms **"inhibitor of lipid metabolism"** refer to any compound leading, either directly or indirectly, at least to a lowering of lipid availability or capability to be used by cell as a source of energy through FA oxidation. In other words, it refers to an inhibitor of any of the enzymes responsible for the synthesis of FA, for the degradation of FA more particularly for the fatty acids oxidation, but also to compounds exposure to which results in an increase of the storage and synthesis of lipids and more particularly FA, or in decrease of the release from storage.

**"Cancer cell sample"** is thought to include any sample which comprises cancer cells from which the subject to be classified is suffering from. In a particular embodiment, "cancer cell sample" is a sample of tumour tissue, i.e. sample from a biopsy or of tumour resection, or even the tumour tissue itself. In another embodiment, said cancer cell sample is a sample from a metastasis, resulting from either a biopsy (e.g., lymph nodes biopsies) or resection. tumour tissue, i.e, sample from a biopsy or of tumour resection, or even the tumour tissue itself. When related to liquid cancers, in other words to liquid tumor, cancer cell sample can also refer to body fluids as the blood, bone marrow or a subfraction thereof as plasma. Except when stated otherwise, "Cancer cell sample" or "cancer sample" have the same signification as defined above and are used interchangeably.

As used herein, **"treatment"** or **"to treat"** include the therapy, the prevention, the prophylaxis, the retardation or the reduction of symptoms provoked by or of the causes of a disease. When related to cancer more particularly includes stopping, stabilizing or slowing down the progression of a disease, or lessening the severity of its symptoms. In other words, it encompasses the eradication of a tumour, decreasing the size of a tumour, stopping or slowing down the development of tumor. The terms "treatment" or "to treat" also encompasses therapy, prevention, prophylaxis, retardation or reduction of symptoms of the disease in a subject who has been subjected to a treatment for a cancer and who, after a remission period, is experiencing or suspected to experience relapse. Also, "treatment" or "to treat" encompass therapy, prevention, prophylaxis, retardation or reduction of symptoms of cancer refractory to current treatments as e.g. chemotherapeutic treatment, immunotherapies, anti-metabolic drugs targeting mitochondrial metabolism, anti-glycolytic therapies.

The terms **"Combination"** or **"combinatorial treatment/therapy"** or **"combinatory treatment"** as used herein designate a treatment of a disease or disorder wherein at least two or more drugs and/or active compounds are co-administered to a subject to cause a biological effect. In a combined therapy according to this invention, the drugs may be administered at the same time, together or separately, or sequentially. Also, they may be administered through different routes and protocols. For example, one of the at least two or more drugs can be formulated to be administered orally and one of the others can be administered intravenously. Although, the at least two or more drugs and/or active compounds might be formulated together, the at least two or more drugs and/or active compounds might also be formulated separately.

As used herein, a named compound or drug is thought to designate the chemical compound or drug as named in the application, as well as any pharmaceutically acceptable salt, hydrate, stereoisomer, racemate, conjugate, pro-drug thereof, of any purity.

The term **"salt"** or **"salts"** refers to a pharmaceutically acceptable and relatively non-toxic, inorganic or organic acid addition salt of a compound of the present invention. Salt selection is now a common standard operation in the process of drug development. Though, most salts of a given active principle are bioequivalents, some may have, among others, increased solubility or bioavailability properties. Regarding compounds which have already been developed and/or approved as a medication, preferred salts for these compounds will be those as already developed and/or approved.

The term **"prodrug"** refers to a precursor of a compound which upon administration, generates said compound upon chemical or enzymatic reaction. Prodrug design is a common standard operation in the process of drug development. Regarding compounds which have already been developed and/or approved as a medication, preferred prodrugs for these compounds, if any, will be those as already developed and/or approved

### Methods of classifying/identifying metabolically reprogramed cancers

As exemplified in the experimental data, RelB has been found to be responsible for reorientation of cancer metabolism through promoting an increase of fatty oxidation, a decrease in lipid storage, a decrease of glycolysis pathway, as well as in the enhancement of OxPhos energetic metabolism. This so-called metabolic reprograming is also responsible for higher cell division rate in cells and resistance to cell death. Accordingly, activation of RelB is found to be associated with a worse survival expectancy in HCC patients. Further, inhibition of RelB or of fatty acid oxidation or uptake is found to be detrimental to the cells exhibiting RelB activation, in either solid or liquid cancer. Accordingly, a first object of the invention is an *in vitro* method for classifying a subject afflicted with a cancer as suffering from a cancer with a, or at risk of, metabolic reprograming towards fatty acids oxidation, said method comprising a step of assaying the activation of RelB in a cancer cell sample from said cancer, wherein detecting said the activation is indicative of, or of the risk of, the metabolic reprograming of said cancer towards fatty acids oxidation. This is of a particular interest in order to purposely and selectively applying fatty acid targeting therapy to cancer without unnecessarily exposing the patient to useless medication and to the risk of selecting otherwise metabolically modified cells with a risk of being highly proliferative.

In an embodiment, RelB activation is detected by evidencing in cellular extract from said cancer cells from said subjects, the presence of activated RelB protein, through for example EMSA combined with supershift which allows the detection of DNA binding of RelB containing complexes (see experimental data). This is of particular interest, as even the presence of a low number of cells presenting activated ReIB, and even one, is sufficient to classify the subject afflicted with said cancer at risk to present a tumor with a metabolic reprograming, as these cells can be selected and amplified because of unsuitable medication.

In another embodiment, RelB activation is detected by measuring an increase of RelB expression at the transcriptional or protein level. In a preferred embodiment, such increase is of at least 1.5, of at least 2, or even of at least 3 at the transcriptional level when compared to other cancer cell known to be not RELB dependent. In another preferred embodiment, such increase is of at least 1.5, of at least 2, or even of at least 3 at the transcriptional level when compared to other cancer cell known to be not RELB dependent. Such increase can be measured by any tools or methods well known from the skilled in the art.

In another embodiment, RelB activation is detected by measuring an increase of RelB expression at the transcriptional or protein level. In a preferred embodiment, such increase is of at least 1.5, of at least 2, or even of at least 3 at the transcriptional level when compared to other cancer cell known to be not RELB dependent. In another preferred embodiment, such increase is of at least 1.5, of at least 2, or even of at least 3 at the transcriptional level when compared to other cancer cell known to be not RELB dependent. Such increase can be measured by any suitable tools or methods well known from the skilled in the art.

In a particular embodiment, RelB activation is detected by measuring an increase or a decrease of at least one gene known to be controlled in such a way when RelB protein pathway is activated. Example of such genes from RelB gene signature is given in WO2021205025.

In another embodiment, RelB activation is detected at the posttranslational level by detecting the phosphorylation of RelB protein either at serine 368, threonine 84, serine 552, serine 573 and/or serine 472. Indeed, several phosphorylation sites have been already characterized on the RelB protein (Baud V & Collares D, 2016). For example, phosphorylation at serine 368 has been shown to be required for NF-κB DNA binding activity, dimerization with other NF-κB subunits (p105/p50, p100/p52), and p100 half-life (Maier H.J. *et al*., 2003). Phosphorylation of threonine 84 and the serine 552 were found to be associated with the induction of RelB degradation by the proteasome in T cell lines (Marienfeld R. *et al*., 2001). Phosphorylation on serine 472 was shown to induce dissociation of RelB from its interaction with the inhibitory protein IκBα and to allow its binding to the promoter of critical migration-associated genes (Authier H. *et al*., 2014). Phosphorylation of RelB proteins can be detected on cancer cell extract from a tested cancer cell sample by any suitable tools or methods well known from the skilled in the art, and antibodies are commercially available. Example of antibodies for detecting RelB phosphorylation at the above phosphorylation sites can be selected amongst those listed in Table 1 below.

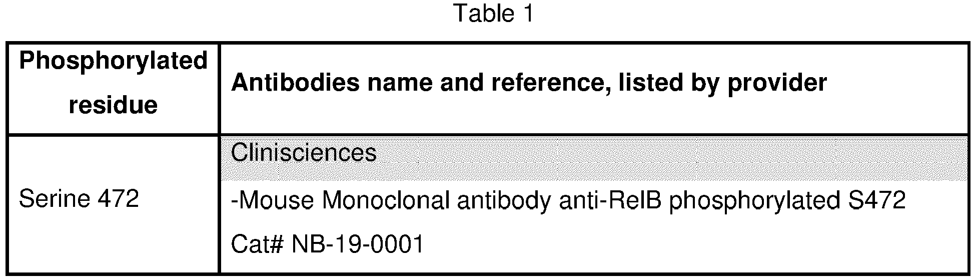

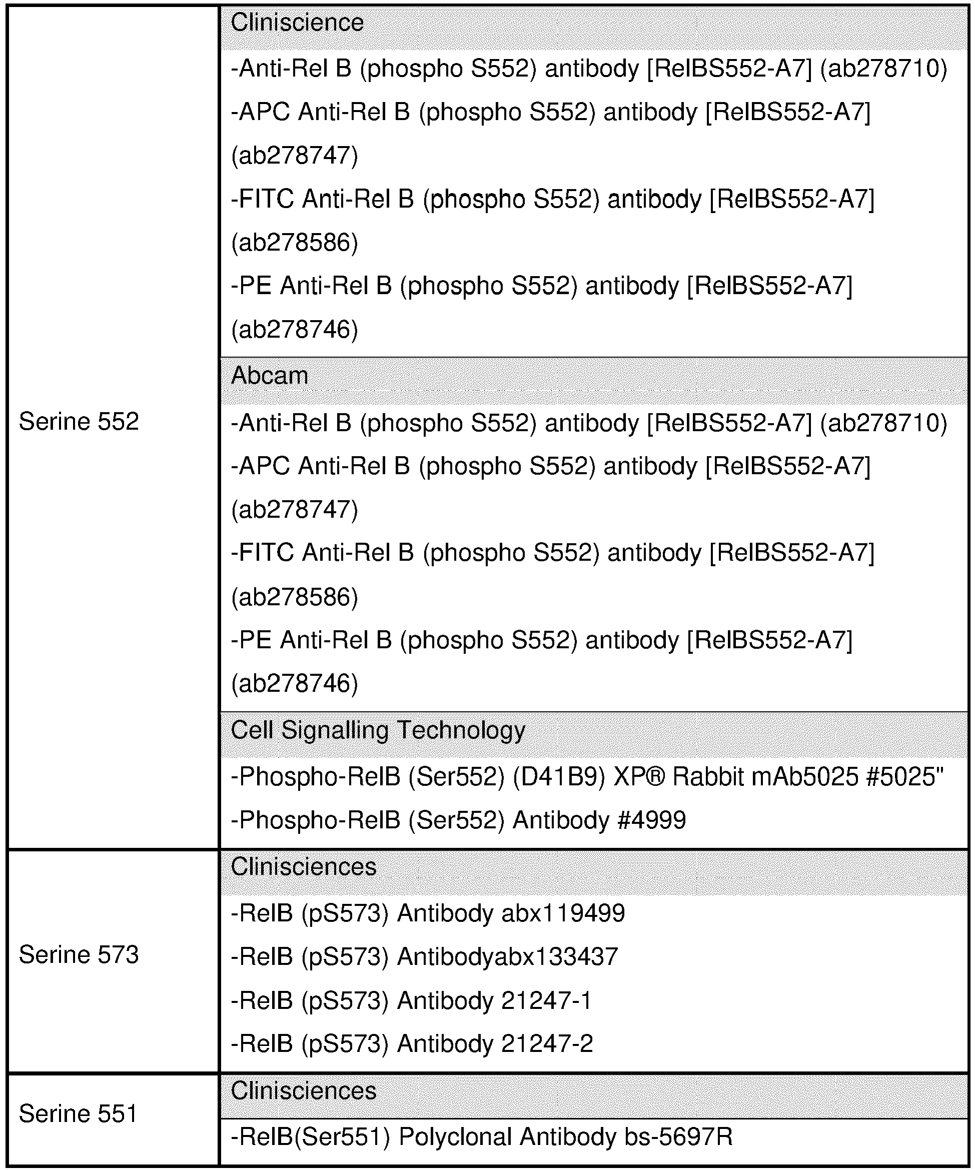

As detailed in the experimental section, a ReIB gene signature related to the metabolic reprograming of cancer towards fatty acids oxidation has been identified by the inventor. Also, in a particular embodiment, the method for classifying a subject afflicted with a cancer as suffering from a cancer with a metabolic reprograming towards fatty acids oxidation further comprises determining the expression level of at least one gene selected in the group consisting of CXCL8, NPPB, BAG4, CNTF, SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3 and GPRC6A. More particularly in this embodiment of the method of the invention, detecting an alteration in the expression of a factor of at least 1.2, preferably 1.3, 1.4 even more preferably at least 1.5 is indicative of, or of the risk of, the metabolic reprograming of said tumor towards fatty acids oxidation. Said alteration can be measured by comparing expression level of the tested sample cell for a given gene, with the expression level of said gene in a reference sample, as e.g. a cell line from the same lineage known to not comprising activation of ReIB, or to not display metabolism reprograming. In a preferred embodiment, the method comprises determining the expression level of several of the above listed genes, preferably at least 2, 3, 4, 5, 6, 7, 8, 9, 10 11, 12, 13, 14, 15, 16, 17, 18, 19, 20, 21, 22, 23, 24, 25, 26, 27, or even 28 of the above listed genes. Table 2 below provides the genes of the RelB gene signature related to the metabolic reprograming towards fatty acid oxidation with their reference in the ENSEMBL European database which gather all the sequenced and validated transcripts common in NCBI and EMBL-EBI databases.

**Table 2**

| **Ensembl gene ID** | **Gene name** | **Ensembl gene ID** | **Gene name** |
|---|---|---|---|
| ENSG00000169429 | CXCL8 | ENSG00000196620 | UGT2B15 |
| ENSG00000120937 | NPPB | ENSG00000109424 | UCP1 |
| ENSG00000156735 | BAG4 | ENSG00000124212 | PTGIS |
| ENSG00000242689 | CNTF | ENSG00000100311 | PDGFB |
| ENSG00000100003 | SEC14L2 | ENSG00000005884 | ITGA3 |
| ENSG00000197971 | MBP | ENSG00000173612 | GPRC6A |
| ENSG00000160179 | ABCG1 | ENSG00000163295 | ALPI |
| ENSG00000132470 | ITGB4 | ENSG00000138061 | CYP1B1 |
| ENSG00000182600 | SNORC | ENSG00000005249 | PRKAR2B |
| ENSG00000157765 | SLC34A2 | ENSG00000131370 | SH3BP5 |
| ENSG00000124813 | RUNX2 | ENSG00000171791 | BCL2 |
| ENSG00000140398 | NEIL1 | ENSG00000168918 | INPP5D |
| ENSG00000118402 | ELOVL4 | ENSG00000198099 | ADH4 |
| ENSG00000101290 | CDS2 | ENSG00000118514 | ALDH8A1 |

Preferably, when the expression level of any one of CXCL8, NPPB, BAG4 and CNTF genes is measured as mentioned above, then a decrease is indicative of (or a risk of) metabolic reprograming toward fatty acid oxidation. Preferably, when the expression level of any one of SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D genes is measured as described above, then a decrease is indicative of (or a risk of) metabolic reprograming toward fatty acid oxidation.

In a very particular embodiment, the invention also relates to a method for classifying a subject afflicted with a cancer as suffering from a cancer with a metabolic reprograming towards fatty acids oxidation comprising determining the expression level of at least one gene selected in the group consisting of CXCL8, NPPB, BAG4, CNTF, SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3 and GPRC6A. More particularly in this embodiment of the method of the invention, detecting an alteration in the expression of a factor of at least 1.2, preferably 1.3, 1.4 even more preferably at least 1.5 is indicative of, or of the risk of, the metabolic reprograming of said tumor towards fatty acids oxidation. Said alteration can be measured by comparing expression level of the tested sample cell for a given gene, with the expression level of said gene in a reference sample, as e.g. a cell line from the same lineage known to not comprising activation of ReIB, or to not display metabolism reprograming. In a preferred embodiment, the method comprises determining the expression level of several of the above listed genes, preferably at least 2, 3, 4, 5, 6, 7, 8, 9, 10, 11, 12, 13, 14, 15, 16, 17, 18, 19, 20, 21, 22, 23, 24, 25, 26, 27, or even 28 of the above listed genes. In a preferred embodiment, the method comprises determining the expression level of at least one gene selected in the group consisting of ABCG1, ALDH8A1, ALPI, CDS2, CYP1B1, ELOVL4, PTGIS, PRKAR2B, UGT2B15 and HPRT. More preferably, the method comprises determining the expression level of several of these 10 listed genes, preferably at least 2, 3, 4, 5, 6, 7, 8, 9 or even 10. Preferably, when the expression level of any one of CXCL8, NPPB, BAG4 and CNTF genes is measured as mentioned above, then a decrease is indicative of (or a risk of) metabolic reprograming towards fatty acid oxidation. Preferably, when the expression level of any one of SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D genes is measured as described above, then a decrease is indicative of (or a risk of) metabolic reprograming towards fatty acid oxidation.

In a particular embodiment, the method comprises determining a decrease in the expression of at least one gene, preferably 2, 3 or even 4 genes, selected from the group consisting of CXCL8, NPPB, BAG4 and CNTF.

In another particular embodiment, the method comprises determining an increase in the expression of at least one gene, preferably 2, 3, 4, 5, 6, 7, 8, 9, 10, 11, 12, 13, 14, 15, 16, 17, 18, 19, 20, 21, 22, 23, or even 24 genes, selected from the group consisting of SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D.

In a further particular embodiment, the method comprises determining an increase in the expression of at least one gene, preferably 2, 3, 4, 5, 6, 7, 8, 9, or even 10 genes, selected from the group consisting of ABCG1, ALDH8A1, ALPI, CDS2, CYP1B1, ELOVL4, PTGIS, PRKAR2B, UGT2B15 and HPRT.

In a particular embodiment, the method comprises determining :
- a decrease in the expression of at least one gene selected from the group consisting of CXCL8, NPPB, BAG4 and CNTF, and
- an increase in the expression of at least one gene selected from the group consisting of and the expression level of SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D.

In another particular embodiment the method comprises determining :
- a decrease in the expression of at least one gene selected from the group consisting of CXCL8, NPPB, BAG4 and CNTF, and
- an increase in the expression of at least one gene selected from the group consisting of and the the expression level of ABCG1, ALDH8A1, ALPI, CDS2, CYP1B1, ELOVL4, PTGIS, PRKAR2B, UGT2B15 and HPRT.

Of note, it has been found by the inventors that subject suffering from cancer with cells exhibiting the above gene signature related to the metabolic reprograming of cancer towards fatty acids oxidation have a worse prognostic survival among in comparison with subject where cancer cells do not display such a signature, underlying its importance in disease classification and management.

The measure of the expression level of Rel B and/or any one of the genes of the RelB gene signature related to the metabolic reprograming of cancer toward fatty acid oxidation is preferably performed at the transcriptional level.

A number of techniques, well known from the skilled in the art, are available to detect alterations in the transcriptional expression of genes such as, for example, in a non-limitation way :
- DNA Microarray: A DNA microarray (also commonly known as DNA chip or biochip) is a collection of microscopic DNA spots attached to a solid surface. Scientists use DNA microarrays to measure the expression levels of large numbers of genes simultaneously or to genotype multiple regions of a genome.
- RNAseq: RNA-Seq is a developed approach to transcriptome profiling that uses deep-sequencing technologies. It provides a precise measurement of levels of transcripts and their isoforms (Wang et al, 2009).
- Nanostring: NanoString's nCounter technology is a variation on the DNA microarray and was invented and patented by Krassen Dimitrov and Dwayne Dunaway. It uses molecular "barcodes" and microscopic imaging to detect and count up to several hundred unique transcripts in one hybridization reaction (Geiss et al, 2008).
- RT-MLPA: Multiplex ligation-dependent probe amplification (MLPA) is a variation of the multiplex polymerase chain reaction that permits amplification of multiple targets with only a single primer pair (Schouten et al, 2002).

All these methods (RNAseq, microarray, Nanostring or RT-LMPA) can be used for determining the gene expression of the markers of the invention.

In another particular embodiment of the *in vitro* method for classifying a subject afflicted with a cancer as suffering from a cancer with a metabolic reprograming towards fatty acids oxidation, further to the step of assaying the activation of ReIB, the method comprises a step of determining, in said cell sample, the level of at least one of lipid or group of lipids selected from : Linoleic acid, Phospholipids, Fatty acids, Triglycerides, Monounsaturated fatty acids (MUFAs), Unsaturated fatty acids (UFAs), cholesterol, choline, Ace-choline and O-P choline. More particularly, when an activation of RelB is detected and a decrease in the level of at least one of lipid or group of lipids selected from : Linoleic acid, Phospholipids, Fatty acids, Triglycerides, MUFAs, UFAs, cholesterol is detected, then the cancer is diagnosticated as a metabolically reprogramed toward fatty acid oxidation. Also, when, in said sample cell, an activation of RelB is detected and an increase in the level of at least one of lipid or group of lipids selected from : choline, Ace-choline and O-P choline is detected, then the cancer is diagnosticated as a metabolically reprogramed toward fatty acid oxidation. In a particular embodiment, when in said cancer cell sample wherein an activation of RelB is detected, an increase in the level of group of lipids selected from : choline, Ace-choline and O-P choline is detected and a decrease in the level of the lipids or group of lipids selected from : Linoleic acid, Phospholipids, Fatty acids, Triglycerides, MUFAs, UFAs and cholesterol is detected, then the cancer is diagnosticated as a metabolically reprogramed toward fatty acid oxidation. Said increase and/or decrease being determined upon comparison with the levels of the corresponding metabolite in a reference sample, as e.g. a cell line from the same lineage known to not comprising RelB activation and/or metabolic reprograming toward fatty acid oxidation. The method of the invention is suitable for identifying the metabolic reprograming of any liquid or solid cancer cell, and more particularly those listed above. In a particular embodiment of said *in vitro* method for classifying a subject afflicted with a cancer as suffering from a cancer with a metabolic reprograming towards fatty acids oxidation, the cancer is a hepatic cancer, more preferably a hepatocellular carcinoma. Indeed, experimental data, besides showing the pivotal role of RelB in metabolic reprograming, further show that RelB activation is shown to be of worse prognostic survival for patient suffering from HCC, thereby underlying the importance of identifying metabolic reprograming in this cancer. Further, the presence of an alteration in lipid content as shown herein is found associated with Rel B expression and worse prognostic survival. In another particular embodiment, said cancer is a non-Hodgkin lymphoma, more preferably a DLBCL. Indeed, it is known in the art that DLBCL with an activated RelB are particularly aggressive lymphoma (Eluard et al, 2022). Further, it is shown herein that RelB is responsible for enhancement of Oxphos metabolism and energy homeostasis in these cells, which are particularly sensitive to the inhibition of lipid metabolism when compared to glycolytic cell lines.

### Management of cancer treatment

Identifying metabolic reprograming, or a of a risk for metabolic reprograming, in a cancer cell sample constitutes a valuable information to select appropriate therapy for the patients in such a manner that they will potentially benefit of the most appropriate therapy as soon as possible, which might be different than commonly administered first line therapy. Such patient stratification could therefore allow to avoid loss of time in treatment of the disease, the administration of useless or weakly effective treatments, cellular adaptations and spread of the disease. Also, identifying a cancer as comprising cancerous cells exhibiting metabolic reprograming towards fatty acid oxidation enlarge the available treatment options and let to expect a more effective treatment plan.

RelB activation has been identified by the inventor as responsible for the redirection of the cell metabolism towards fatty acid oxidation and for regulating energy homeostasis in cancer cells.

### Inhibitor of the expression of RelB and/or of the activity of RelB

Accordingly, a second object of the invention relates to an inhibitor of the expression of RelB and/or of the activity of RelB for use in inhibiting or preventing metabolic reprograming towards fatty acids oxidation in a cancer cell. Indeed, such a therapeutic use in the treatment plan of a patient is of interest in order to avoid or prevent cellular adaptations and/or the selection of metabolic reprogramed cells leading to the relapse and/or the emergence of a refractory disease.

This therapeutic use is further of an outmost interest in the treatment of cancer wherein such a metabolic reprograming is detected. Indeed, inhibition of RelB expression is shown as lowering growth capacity/viability of the cells with metabolically reprogramed cells towards fatty acid oxidation and/or in which RelB is activated ; also inhibiting RelB expression results in lowering RelB protective effect against cell death ; further, RelB expression level is shown correlated with a worse life duration expectancy in diseased subjects. Also, in an embodiment, the invention relates to an inhibitor of the expression of RelB and/or of the activity of RelB for use in inhibiting metabolic reprograming towards fatty acids oxidation in a cancer cell showing a metabolic reprograming towards fatty acid oxidation. In other words, an embodiment of the invention relates to an inhibitor of the expression of RelB and/or of the activity of RelB for use in treating cancer wherein a metabolic reprograming toward fatty acid oxidation is detected, notably, but not only, through the implementation of the method according to the first object of the invention, in any of its embodiments. Particularly, in a preferred embodiment, the cancer cell sample from said cancer an activation of RelB is detected.

More particularly, in an embodiment, in the cancer cell sample from said cancer, an activation of RelB is detected and/or an alteration of the expression of at least one gene selected from RELB, CXCL8, NPPB, BAG4, CNTF, SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D as exposed above.

In another embodiment, in the cancer cell sample from said sample, an activation of RelB is detected and/or levels of lipid metabolites are found modified as stated in table 3 below, in comparison with the levels of the corresponding metabolite in a reference sample, as e.g. a cell line from the same lineage known to not comprising RelB activation and/or metabolic reprograming towards fatty acid oxidation.

**Table 3**

| **Insoluble fraction** | |
|---|---|
| **Linoleic acid** | decrease |
| **Phospholipids** | decrease |
| | |
| **Triglycerides** | decrease |
| **Monounsaturated fatty acids (MUFAs)** | decrease |
| **Unsaturated fatty acids (UFAs)** | decrease |
| **Fatty acids other than UFAs and MUFAs** | decrease |
| **cholesterol** | decrease |

| **Soluble fraction** | |
|---|---|
| **choline** | increase |
| **Ace-choline** | increase |
| **O-P choline** | increase |

Preferably, when an increase of a level of a lipid metabolite of table 3 is measured, said increase is at least of 1.5, 2, 3, 4, 5 even at least 10 times higher than the level of the corresponding metabolite in a reference sample. Preferably, when a decrease of a level of lipid metabolite of table 3 is measured, said decrease is at least of 1.5, 2, 3, 4, 5, even at least 10 times higher than the level of the corresponding metabolite in a reference sample.

Any means known for inhibiting in the art to inhibit the expression of RelB and/or of the activity of RelB provides a valuable candidate as compound for inhibiting activation of ReIB.

In an embodiment, the inhibitor of the expression of RELB and/or of the activity of RELB for use in inhibiting metabolic reprograming towards fatty acids oxidation in a cancer cell comprises at least:
- an antisense nucleotide or a siRNA or a shRNA designed against RelB gene, and/or
- an anti-RELB antibody, fragment or derivative thereof.

As shown in the experimental section, an inhibitor of the expression of RelB can be an antisense nucleotide which is designed to extinguish expression of the RelB gene. Antisense oligonucleotides therapies are currently marketed and approved by several national health agencies. Antisense nucleotide can be designed according to methods well known in the art. Also, an antibody, preferably a monoclonal antibody, directed against RelB and therefore inhibiting RelB DNA binding activity when complexed thereto, constitutes a particularly suitable tools as inhibitors of the activity of ReIB. Also in a preferred embodiment, the at least one inhibitor of the activity of RelB is a monoclonal antibody directed against ReIB. More preferably said antibody is a monoclonal antibody directed against a phosphorylated ReIB. In a particular embodiment, said inhibitor can be a fragment or a derivative of said antibody (which for example enters more easily inside the cells), provided it retains a binding activity and an inhibitory activity towards RelB activation. More particularly said antibody specifically binds phosphorylated RelB protein at serine 368, threonine 84, serine 552, serine 573 and/or serine 472. Even more particularly said antibody specifically binds specifically serine 472 and comprises a heavy chain and/or light chain as specified in table 4 below. Even more particularly said antibody specifically binds specifically serine 472 and comprises a heavy chain and/or light chain with H-CDR and/or L-CDR sequences as specified in table 4 below.

**Table 4**

| | |
|---|---|
| anti-pSer 472 RelB antibody IgG Domains | Protein Sequence |
| VH | EVQLVETGGGLVQPKGSLKLSCAVSGFTFNTDAMNWVR QAPGKGLEWVARMRSKSNNYATYYADSVKDRCTISRDDS QSMFYLQMNNLKTEDTAMYYCVYGGAMDYWGQGTSVTV SS (SEQ ID NO:2) |
| H CDR1 | TDAMN (**SEQ ID NO:3**) |
| H CDR2 | RMRSKSNNYATYYADSVKD (**SEQ ID NO:4**) |
| H CDR3 | VYGGAMDY (**SEQ ID NO:5**) |
| VL | DIVLTQSPASLVVSLGQRATISCRASKSVYTSGYSYMHWF QQKPGQPPKLLIKYASNLQSGVPARFSGSGSGTDFTLNIH PVEAEDTATYYCQHNWEIPLTFGAGTKLELK (SEQ ID NO:6) |
| L CDR1 | RASKSVYTSGYSYMH (**SEQ ID NO:7**) |
| L CDR2 | YASNLQS (**SEQ ID NO:8**) |
| L CDR3 | QHNWEIPLT (**SEQ ID NO:9**) |

A "monoclonal antibody", as used herein, means an antibody arising from a nearly homogeneous antibody population. The individual antibodies of a population are identical except for a few possible naturally-occurring mutations which can be found in minimal proportions. In other words, a monoclonal antibody consists of a homogeneous antibody arising from the growth of a single cell clone (for example a hybridoma, a eukaryotic host cell transfected with a DNA molecule coding for the homogeneous antibody, a prokaryotic host cell transfected with a DNA molecule coding for the homogeneous antibody, etc.) and is characterized by heavy chains of one and only one isotype and subtype, and light chains of only one type. Monoclonal antibodies are highly specific and are directed against a single epitope of an antigen. Monoclonal antibodies may be produced by a single clone of B cells or "hybridoma". Monoclonal antibodies may also be recombinant, i.e. produced by protein engineering.

Suitable monoclonal antibodies may be produced in a hybridoma cell line according to the well-known technique of Kohler and Milstein, 1975; Kohler and Milstein, 1976; see also, CURRENT PROTOCOLS IN MOLECULAR BIOLOGY, Ausubel et al (1989). Suitable fragments of an antibody inhibitor of the activity of RelB can be selected from Fab, Fab', F(ab')2, scFv, dsFv, ds-scFv, dimers, minibodies, diabodies, multimers thereof or bispecific antibody fragments. Antibodies can be fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, dsFv, ds-scFv, dimers, minibodies, diabodies, bispecific antibody fragments and other fragments can also be synthesized by recombinant techniques. For example, scFvs, comprise the variable regions of the immunoglobulin heavy and light chain, covalently connected by a peptide linker. These "antibody fragments" are rather small in comparison with antibodies and generally retain specificity and affinity for antigen in a single polypeptide. Also, any combination of part(s) or of fragment(s) of antibodies as described above provided that it retains sufficient affinity constant and/or dissociation constant for RelB protein.

Particularly preferred antibodies are "single chain antibody" or sdAb, or related molecules, which refers to the single heavy chain variable domain (VHH) of antibodies of the type that can be found in camelids which are naturally devoid of light chains. Such single domain antibodies are also named nanobodies. They represent the smallest antibody fragments (around 14 kDa) able to preserve the binding affinity and specificity of the original whole antibody and they are appreciated for their structural stability and their simple engineering into reagents suitable for *in vitro* and *in vivo* applications (de Marco (2020)). They can be obtained by classical immunization of animals known to produce such antibodies, e.g. camelids or cartilaginous fishes with the desired antigen and subsequent isolation of the mRNA coding for heavy-chain antibodies. They can be also produced and purified using genetic engineering as reviewed in de *Marco* (2020). Their relatively low molecular mass leads to a better permeability in tissues, and to a short plasma half-life making sdAb of a particular interest in therapeutic applications. Capacity of said antibodies, fragment or derivative thereof, to inhibit DNA binding activity of RelB can be assayed by any method known in the art. For example, a well-known method is ELISA (Methods in Enzymology, Volume 70, Pages 3-525 (1980)) or the Electrophoretic Mobility Shift Assay as described in the experimental Part.

Basically, any type of cancer is susceptible to be subjected metabolically reprogramed toward fatty acid oxidation. Also, in an embodiment, the inhibitor of the expression of RelB and/or of the activity of RelB is for use in inhibiting metabolic reprograming towards fatty acids oxidation in a cancer cell is selected from breast, prostate, pancreatic cancers, glioma, glioblastoma, ovarian, endometrial, skin and liver cancer, a Hodgkin lymphoma, a diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL) or leukemia. Liver cancer or DLBCL are particularly preferred. In an embodiment, the inhibitor of the expression of RelB and/or of the activity of RelB is for use in treating cancer selected from breast, prostate, pancreatic cancers, glioma, glioblastoma, ovarian, endometrial, skin and liver cancer, a Hodgkin lymphoma, a diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL) or leukemia, wherein a metabolic reprograming towards fatty acid oxidation is detected notably, but not only, through the implementation of the method according to the first object of the invention, in any of its embodiments. Liver cancer or DLBCL are particularly preferred.

### Inhibitor of lipid metabolism

As shown in the experimental section, in cancer cells showing an activation of ReIB, RelB protective effect against cell death is abolished when exposing these cells to an inhibitor of lipid metabolism. Such cells are thus particularly dependent on fatty acids as a source of energy, and, accordingly, are found to be particularly sensitive to inhibition of lipid metabolism.

Also in a third object, the invention relates to an inhibitor of lipid metabolism for use in treating or preventing a cancer showing an activated ReIB, in other words, a cancer for which a cancer cell sample shows an activation of ReIB.

In an embodiment, the invention relates to an inhibitor of lipid metabolism for use in treating a cancer showing an activated RelB and showing a metabolic reprograming towards fatty acid oxidation. More particularly, an embodiment of the invention relates to an inhibitor of lipid metabolism for use in treating a cancer showing an activated RelB and an alteration of the expression of at least one gene selected from CXCL8, NPPB, BAG4, CNTF, SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D, and measured as described above. In an even more particular embodiment, said cancer shows a decrease in the expression of at least one selected from CXCL8, NPPB, BAG4 and CNTF genes. In another particular embodiment, said cancer shows an increase in the expression of at least one gene selected from SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D.

Even more particularly, in said cancer showing an activated ReIB, in the cancer cell sample from said sample, levels of lipid metabolites are found modified as stated in table 3, in comparison with the levels of the corresponding metabolite in a reference sample as exposed above.

Suitable inhibitors of lipid metabolism can be selected from, for example, an inhibitor of fatty acid synthase (FASN), an inhibitor of ATP citrate lyase (ACLY), an inhibitor of acetyl-CoA carboxylase (ACC), an inhibitor of fatty acid synthase (FASN) or FAS), and inhibitors of acyl-CoA synthetases (ACS, aka fatty acid-CoA ligases), an inhibitor of carnitine palmitoyl transferase 1 (CPT1), inhibitors of monoacylglycerol lipase (MAGL), inhibitors of sterol regulatory element-binding protein 1 and/or 2, or a combination thereof.

Examples of suitable inhibitors of lipid metabolism are listed in table 5 below.

**Table 5**

| **Compounds Inhibitors of lipid metabolism** | **CAS number** | **Possible target** |
|---|---|---|
| soraphen-A | 122547-72-2 | ACC |
| TOFA (5-(tetradecyloxy)-2-furoic acid) | 54857-86-2 | |
| A-769662 | 844499-71-4 | |
| metformin | 657-24-9 or 1115-70-4 | |
| AICAR ([(2R,3S,4R,5R)-5-(5-amino-4-carbamoylimidazol-1-yl)-3,4-dihydroxyoxolan-2-yl]methyl dihydrogen phosphate | 3031-94-5 | |
| SB-204990 | 154566-12-8 | ACY |
| LY294002 | 154447-36-6 | |
| triacsin C | 76896-80-5 | ACS |
| thiazolidinediones (TZDs) | na | |
| TCD-717 | 1437763-71-7 | CKα |
| MN58b | 203192-01-2 | |
| 1,2,3, benzene-tricarboxylate | 569-51-7 | CIC |
| etomoxir | 82258-36-4 | CPT1 |
| ranolazine | 95635-55-5 | |
| ST1326 | 250694-07-6 | |
| glyburide | 10238-21-8 | |
| perhexiline | 6621-47-2 | |
| cerulenin | 17397-89-6 | FASN |
| C75 | 218137-86-1 | |
| TVB-2640 | 1399177-37-7 | |
| TVB-3166 | 1533438-83-3 | |
| orlistat | 96829-58-2 | |
| flavonoids | na | |
| epigallocatechin-3-gallate | 298700-58-0 | |
| platensimycin | 835876-32-9 | |
| GSK837149A | 13616-29-0 | |
| JZL-184 | 1101854-58-3 | MAGL |
| BZ36 (6-[4-(2-Bromo-5-methoxybenzoyl)-piperazin-1-yl]-N-phenylpropyl-nicotinamide) | na | SCD |
| A939572 | 1032229-33-6 | |
| fatostatin | 125256-00-0 / 298197-04-3 | SREBP1 and 2 |
| FGH10019 | 1046045-61-7 | |
| betulin | 473-98-3 | |
| 25-Hydroxycholesterol | 2140-46-7 | |

Thiazolidinediones refer to a well-known family of drugs usually used in the treatment of diabetes mellitus type 2 but which have been found to inhibit ACS, which comprises pioglitazone (CAS : 111025-46-8), rosiglitazone (CAS : 122320-73-4), lobeglitazone (CAS : 607723-33-1), ciglitazone (CAS : 74772-77-3), darglitazone (CAS : 141200-24-0), englitazone (CAS: 109229-58-5), netoglitazone (CAS : 161600-01-7), rivoglitazone (CAS : 185428-18-6), troglitazone (CAS: 97322-87-7), balaglitazone (CAS : 199113-98-9), AS-605240 (CAS : 648450-29-7) or any salt or prodrug thereof.Flavonoids are well-known chemical compounds that display numerous biological actions, and which have been found effective in inhibiting FASN. Preferred flavonoids are selected from luteolin (CAS : 491-70-3), quercetin (CAS : 117-39-5), kaempferol (CAS : 520-18-3), morin (CAS : 480-16-0), fisetin (CAS : 528-48-3), myricetin (CAS : 529-44-2), or salts or prodrugs thereof. Accordingly, in a particular embodiment, said inhibitor of lipid metabolism is selected from at least one compound from the group of soraphen-A, TOFA (5-(tetradecyloxy)-2-furoic acid), A-769662, metformin, AICAR ([(2R,3S,4R,5R)-5-(5-amino-4-carbamoylimidazol-1-yl)-3,4-dihydroxyoxolan-2-yl]methyl dihydrogen phosphate, SB-204990, LY294002, triacsin C , a thiazolidinedione, e.g. as cited above, TCD-717, MN58B, 1,2,3, benzene-tricarboxylate, etomoxir, ranolazine, ST1326, glyburide, perhexiline, cerulenin, C75, TVB-2640, TVB-3166, orlistat, a flavonoid e.g. as one of those specified above, epigallocatechin-3-gallate, platensimycin, GSK837149A, JZL-184, BZ36 (6-[4-(2-Bromo-5-methoxy-benzoyl)-piperazin-1-yl]-N-phenylpropyl-nicotinamide), A939572, fatostatin, FGH10019, betulin, and 25-hydroxycholesterol, or any pharmaceutically acceptable salts or prodrugs thereof, or a combination thereof. Targeting lipid metabolism in several points is of particular interest in order to subvert rapid proliferation of cancer cells and to increase sensitivity to cell death with an activation of ReIB. Indeed, as exemplified, these cells are of an increase growth rate and resistance to cell death. Also, in a preferred embodiment the inhibitor of lipid metabolism for use according to the invention refers to a combination of at least two different compounds responsible for the inhibition of at least two different mechanisms implied in lipid metabolism selected from enzymes responsible for i) the synthesis of FA, ii) for the degradation of FA more particularly for the fatty acids oxidation, iii) the storage of lipids and more particularly FA, or iv) for the release from storage. In another preferred embodiment, the inhibitor of lipid metabolism for use according to the invention refers to a combination of at least two compounds selected from soraphen-A, TOFA (5-(tetradecyloxy)-2-furoic acid), A-769662, metformin, AICAR ([(2R,3S,4R,5R)-5-(5-amino-4-carbamoylimidazol-1-yl)-3,4-dihydroxyoxolan-2-yl]methyl dihydrogen phosphate, SB-204990, LY294002, triacsin C , a thiazolidinedione, e.g. as specified above, TCD-717, MN58B, 1,2,3, benzene-tricarboxylate, etomoxir, ranolazine, ST1326, glyburide, perhexiline, cerulenin, C75, TVB-2640, TVB-3166, orlistat, a flavonoid, epigallocatechin-3-gallate, platensimycin, GSK837149A, JZL-184, BZ36 (6-[4-(2-Bromo-5-methoxybenzoyl)-piperazin-1-yl]-N-phenylpropyl-nicotinamide), A939572, fatostatin, FGH10019, betulin, and 25-hydroxycholesterol, or any pharmaceutically acceptable salt or prodrugs thereof. In an even more preferred embodiment, the inhibitor of lipid metabolism for use according to the invention refers to a combination of at least two compounds selected from soraphen-A, TOFA (5-(tetradecyloxy)-2-furoic acid), A-769662, metformin, AICAR ([(2R,3S,4R,5R)-5-(5-amino-4-carbamoylimidazol-1-yl)-3,4-dihydroxyoxolan-2-yl]methyl dihydrogen phosphate, SB-204990, LY294002, triacsin C , a thiazolidinedione e.g. as specified above, TCD-717, MN58B, benzene-tricarboxylate analog (BTA), etomoxir, ranolazine, ST1326, glyburide, perhexiline, cerulenin, C75, TVB-2640, TVB-3166, orlistat, a flavonoid, epigallocatechin-3-gallate, platensimycin, GSK837149A, JZL-184, BZ36 (6-[4-(2-Bromo-5-methoxy-benzoyl)-piperazin-1-yl]-N-phenylpropyl-nicotinamide), A939572, fatostatin, FGH10019, betulin, and 25-hydroxycholesterol, or any pharmaceutically acceptable salt or prodrugs thereof, said at least two compounds targeting a different target as specified in table 5.

Inhibitors of lipids metabolism for use according to the invention are suitable for any kind of cancer wherein activation of RelB is detected, more particularly said cancer is selected from breast, prostate, pancreatic cancers, glioma, glioblastoma, ovarian, endometrial, skin and liver cancer, a Hodgkin lymphoma, a diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL) or leukemia. Liver cancer or DLBCL are particularly preferred.

### Combinatory treatments

Targeting cancer cells at multiple points of their metabolism is of the outmost interest in order to subvert rapid proliferation, resistance to cell death and to prevent cellular adaptations and/or the selection of metabolic reprogramed cells leading to the relapse and/or the emergence of a refractory disease. Further, RelB activation is shown herein to be responsible for the enhancement of the oxidative phosphorylation (OxPhos) and metabolic reprograming towards fatty acid; also, combinatory therapies in cancer with a RelB activation could comprise :
- the inhibitor of lipid metabolism for use as described above in any of its embodiments, and
- at least one different antiglycolytic compound as listed in table 6, and/or
- at least one different glutamine metabolism inhibitor as listed in table 6, and/or
- at least one different inhibitor of mitochondrial electron transport chain as listed in table 6,
or salts, or prodrugs thereof.

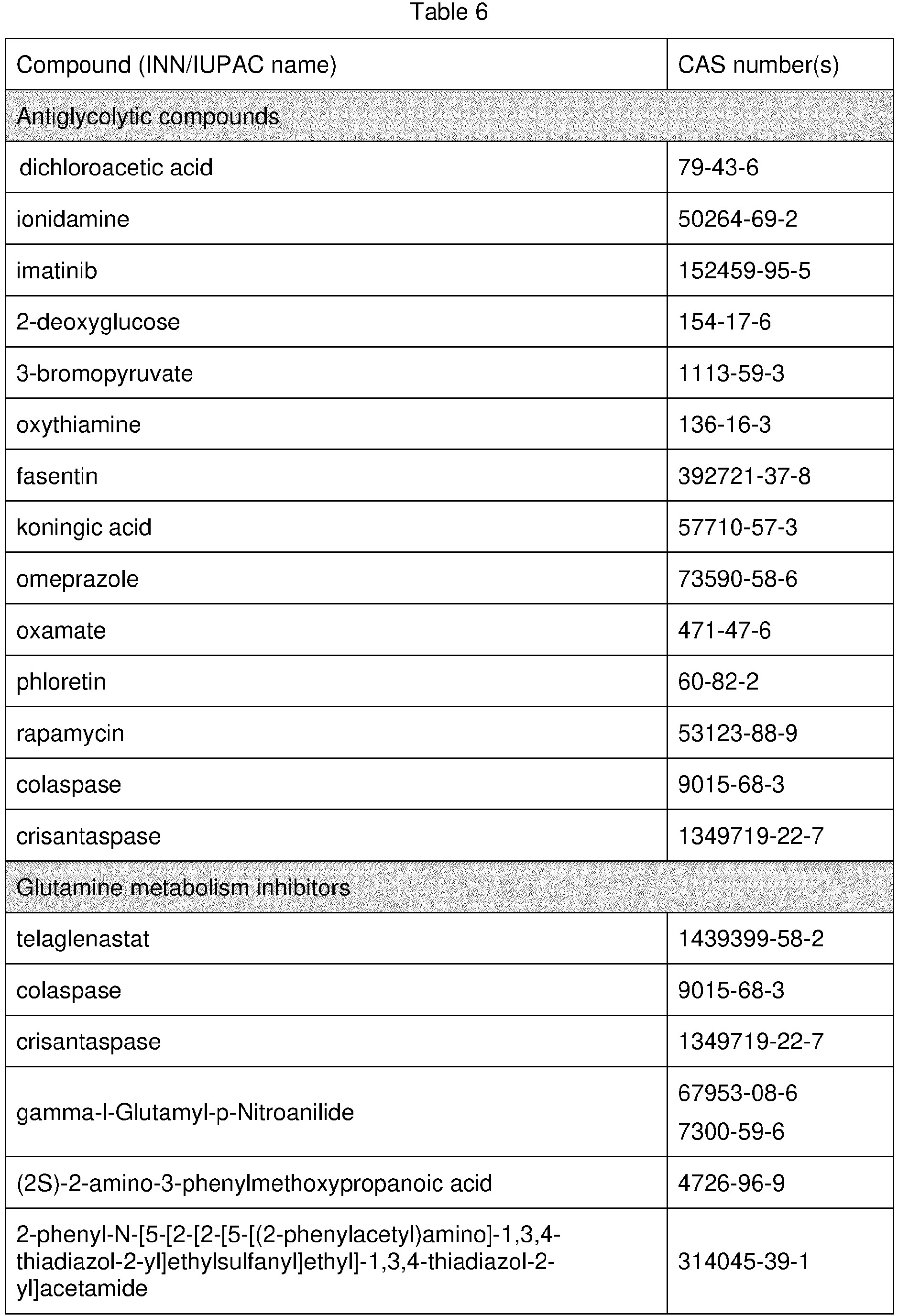

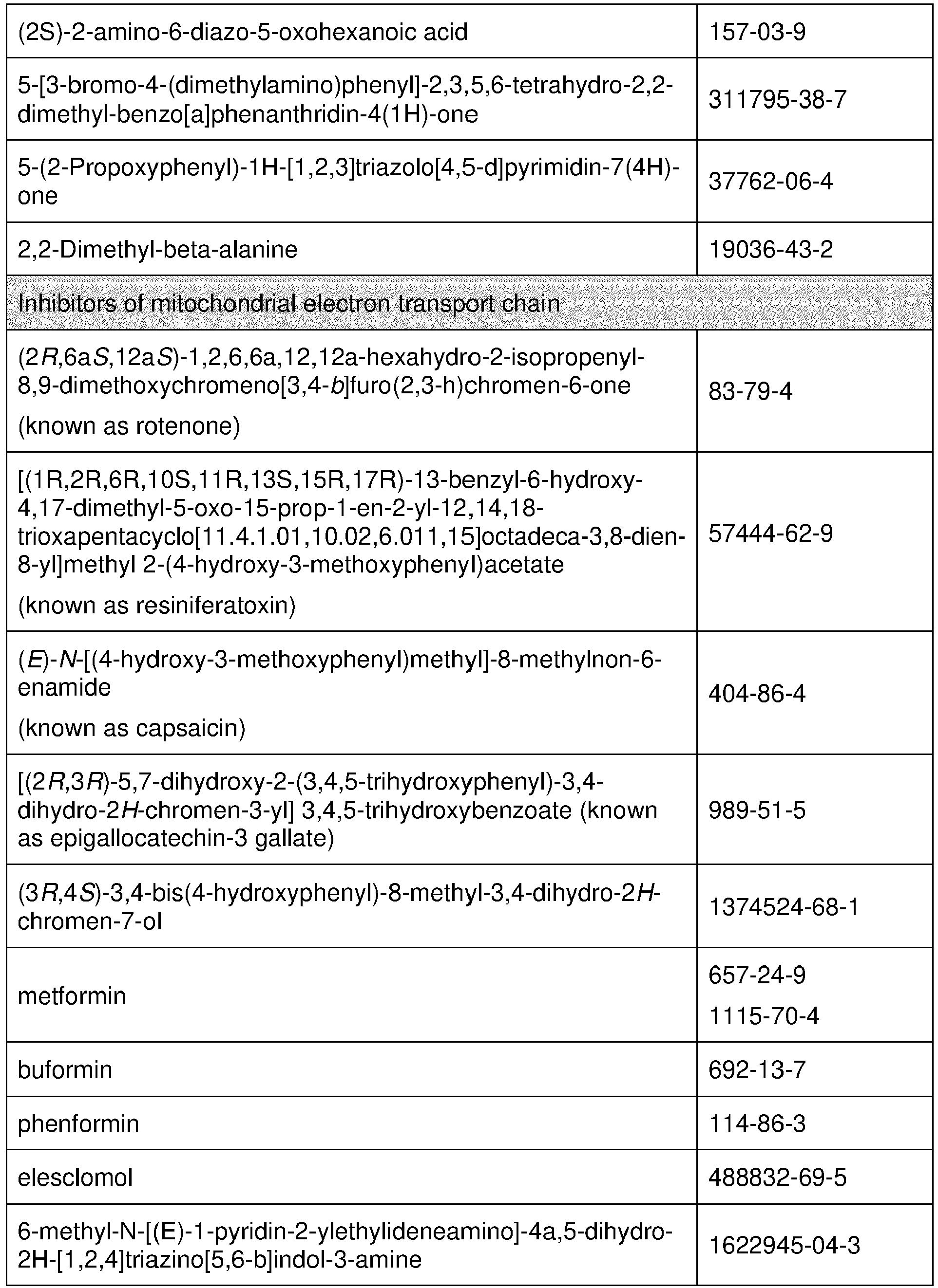

Compounds belong to several categories of table 6, in other words being inhibitors of several of glycolysis, mitochondrial electron transport chain or glutamine metabolism are particularly preferred. This is the case, for example of L-asparaginase (BAN: crisantaspase or colaspase) which displays inhibiting activities for both glycolysis and glutamine metabolism. Metformin is also found as inhibiting mitochondrial electron transport chain and enzyme ACC (therefore inhibiting lipid metabolism). Therefore, metformin and/or L-asparaginase are accordingly particularly preferred to be used in a combinatory treatment according to the invention. As shown in the experimental section, RelB activation being responsible for the metabolic reprograming towards fatty acid oxidation and enhancement of OxPhos, targeting both fatty acid metabolism and mitochondrial metabolism is of particular interest. Also, in a further embodiment of the inhibitor of lipid metabolism for use in treating cancer showing activation of RelB in any of the above embodiments in a combination with at least one mitochondrial metabolism inhibitor which comprises at least one glutamine metabolism inhibitor, for example selected from those listed in table 6, preferably at least one asparaginase (as a colaspase or crisantaspase or derivative thereof), and/or at least one inhibitor of mitochondrial electron transport chain, for example selected from those listed in table 6, preferably metformin.

In a more particular embodiment, the invention relates to an inhibitor of lipid metabolism as described above in a combination with metformin and/or asparaginase for use in treating cancer showing activation of ReIB. In an even more particular embodiment, said inhibitor of lipid metabolism is an inhibitor of fatty acid oxidation as, for example CTP1 inhibitors, in a combination with metformin and/or asparaginase for use in treating cancer, preferably DLBCL.

Further, as shown in the experimental section, inhibiting RelB abolishes RelB protective effect against induced cell death. Also, inhibiting the expression and/or the activity of RelB and/or inhibiting lipid metabolism in cancer cells showing an activation of RelB leads to sensitization of said cells. Also, a particular object of the invention relates to an inhibitor of lipid metabolism as described above for use in sensitizing cancer cells showing an activation of RelB to chemotherapeutic agents. Also, another particular object of the invention relates to an inhibitor of the expression of RelB and/or of the activity of RelB for use in sensitizing cancer cells showing an activation of RelB to chemotherapeutic agents.

A particular object of the invention relates to a method of treating a cancer metabolically reprogramed towards fatty acids oxidation comprising :
a. assaying the activation of RelB in a cancer cell sample from said cancer,
b. if an activation of RelB is detected then administering to the subject suffering from who the cancer cell sample :
   - an inhibitor of the expression of RelB and/or of the activity of RelB as detailed above, and/or
   - an inhibitor of lipid metabolism as detailed above.

In a particular embodiment, said method further comprises determining the expression level of at least one of the genes of the RelB gene signature related to the metabolic reprograming towards fatty acid oxidation. If an activation of RelB is detected as well as an alteration of the level of at least one of the genes of the RelB gene signature related to the metabolic reprograming towards fatty acid oxidation, then proceeding to step b of administering to the subject from who the cancer cell sample :
- an inhibitor of the expression of RelB and/or of the activity of RelB as detailed above, and/or
- an inhibitor of lipid metabolism as detailed above.

In another particular embodiment, said method further comprises determining in said cell sample, the levels of at least one of lipid or group of lipids selected from : Linoleic acid, Phospholipids, Fatty acids, Triglycerides, Monounsaturated fatty acids (MUFAs), Unsaturated fatty acids (UFAs), cholesterol, choline, Ace-choline and O-P choline. More particularly, when an activation of RelB is detected and
- a decrease in the level of at least one of lipid or group of lipids selected from : Linoleic acid, Phospholipids, Fatty acids, Triglycerides, Monounsaturated fatty acids, Unsaturated fatty acids, cholesterol is detected, or
- an increase in the level of at least one of lipid or group of lipids selected from : choline, Ace-choline and O-P choline is detected,
then administering to the subject suffering from who the cancer cell sample :
- an inhibitor of the expression of RelB and/or of the activity of RelB as detailed above, and/or
- an inhibitor of lipid metabolism as detailed above.

In a particular embodiment of said method, the step b further comprises the administration to the subject of at least one compound as listed in table 6 selected from :
- at least one antiglycolytic compound,
- at least one least one inhibitor of glutamine metabolism, and/or
- at least one Inhibitor of mitochondrial electron transport chain.

Further aspects and advantages of the invention will be disclosed in the following examples, which should be considered as illustrative.

### EXAMPLES

### MATERIAL AND METHODS

### 1. Antibodies and Reagents

The antibodies were purchased from Santa Cruz Biotechnology (ReIB C19, RelA C20), and Merck (β-actin). Oleic acid, palmitic acid, 4-bromocrotonic acid (4-BCA), C75, and Oil Red O were purchased from Merck; the CellTiter-Glo^{®} Luminescent Cell Viability Assay kit and DAPI were purchased from Promega and Molecular Probes, respectively. The SYK inhibitor R406 was purchased from Santa Cruz Biotechnology. Metformin, 2-deoxy-D-glucose (2-DG) were purchased from Merck, and L-asparaginase (^{®}Kidrolase) was a gift from Isabelle Madelaine-Chambrin and Nathalie Jourdan (Service de Pharmacie Centrale, Hôpital Saint-Louis, France).

### 2. Cell lines and culture conditions

### Human DLBCL cell lines

MD901 and SUDHL-4 DLBCL cell lines were obtained from Jose Angel Martinez-Climent (Centro de Investigación Medica Aplicada, Pamplona, Spain). Cells were grown in RPMI-1640 Glutamax medium supplemented with 10% heat-inactivated fetal bovine serum (HyClone), 100 U/mL penicillin, and 100 mg/mL streptomycin (Life Technologies).

### Human Hepatocarcinoma (HCC) cell lines

Huh7 HCC cell line and genetically engineered derivatives targeting RelB expression levels (knock-down of RelB expression: Huh7 shRNA human RelB & Huh7 shRNA control; RelB overexpression : Huh7 cDNA human WT Rel & Huh7 cDNA control) were grown in MEM culture medium supplemented with 10 % of fetal bovine serum (FBS), 100 U/mL penicillin, and 100 mg/mL streptomycin, 25mM Hepes Buffer, 1mM of sodium pyruvate, 1 % of non-essential amino-acids (NEAA), which stands for standard culture medium. Huh7 genetically engineered cell lines were only used at passage P1 to P6. Cells passaging occurred twice a week to maintain a condition of 75 % confluence.

To evaluate the effect of fatty acid treatment, Huh7 cells cultured in FBS-free medium for 5 hours were treated for 16 hours in FBS-free medium supplemented with various concentration (50 to 300 µM) of either oleic or palmitic acid along with 1% of free fatty acid bovine serum albumin.

### Lentiviral Production and Transduction

Production of infectious recombinant lentiviruses was performed by transient transfection of 293T cells as previously described (Kieusseian et al 20006). For infections, cells were incubated overnight with recombinant lentiviruses. An equal amount of fresh culture medium was added 24h later and after 48h, cells were washed and seeded in fresh culture medium. GFP positive cells were sorted with FACSAria^{™} sorter (Becton Dickinson).

### 3. Measurement of intracellular ATP content

Intracellular ATP content was measured using a luminescence ATP detection assay kit (CellTiter-Glo^{®} Luminescent Cell Viability Assay, Promega) according to the manufacturer's instructions.

DLBCL cell experiments : Briefly, around 3 x 10⁴ cells were seeded in 100µL of RPMI-1640 Glutamax medium supplemented with 10% FBS (96 well plates). Cells in triplicate were either left untreated or treated for 30 min in presence or not of 20mM 2-deoxy-D-glucose (2-DG) to inhibit glycolysis. At the end of the incubation time, the ATP content was measured by adding 100 µL of CellTiter-Glo reaction mix to each well (final volume of 200µL) for 10 min. The luminescence signal was recorded with a microplate reader (Centro LB 960microplate luminometer, Berthold Technologies). Raw data (Relative Luminescence Units) were analyzed using Microsoft Excel software. The difference between total ATP content and ATP produced under 2-DG treatment results in glycolytic ATP.

Huh7 cells experiments : Briefly, around 4 000 cells were seeded in 96 wells plates in either complete medium or in fatty acid treatment conditions (see fatty acid treatment). 48 hours post-seeding, intracellular ATP content is measured according to the manufacturer's instructions as described above, and adjusted to the number of cells counted the same day in a separate well. Cellular ATP content is obtained by dividing the relative luminescence units (RLU) by the associated cell number (RLU/cell).

### 4. Measurement of mitochondrial transmembrane potential (ΔΨm)

For determination of mitochondrial transmembrane potential (ΔΨm), cells were stained with 100nM of Tetramethyl rhodamine methyl ester (TMRM) (Molecular Probes) in RPMI-1640 Glutamax medium for 30 min at room temperature. Cells were evaluated by cytometric analysis as above. During apoptosis, the permeabilization of the mitochondrial outer membrane leads to loss of mitochondrial transmembrane potential (ΔΨm) corresponding to TMRM low/DAPI neg cells.

### 5. Cell apoptosis/Cell death

### Annexin V binding assay - DLBLC cells

DLBCL cells were harvested and washed twice with cold PBS. Cells were resuspended in 1X binding buffer containing Annexin V/APC (BD Biosciences Pharmingen) and 4',6-diaminidino-2-phenylindole (DAPI, Molecular Probes) for 15 min at room temperature. The samples were subjected to cytometric analysis with a MACSQuant Analyzer 10 Flow Cytometer (Miltenyi Biotec) and the data were analyzed using the Flowjo v10.2 software.

### DAPI staining- Huh7 cells

Huh7 cells were stained with 4', 6-diamidino-2-phenylindole (DAPI). Then, images were acquired for DAPI positive cells by fluorescent microscopy (magnification: 20X) on at least 6 different fields combined with one acquisition under white light to quantify the total number of cells in the same field. Quantification was carried out by image J software. Cell death is expressed in percentage (number of DAPI positive cells / total number of cells per field) *100). To evaluate the pro-survival benefit of mitochondrial fatty acid oxidation (FAO), this program was inhibited using 4-bromocrotonic acid (4-BCA) which irreversibly inhibits mitochondrial β-oxidation of both long- and short-chain fatty acids.

### Cell Viability Assay-DLBCL and Huh7 cells

Cell viability was assessed using trypan blue dye exclusion.

### 6. Electrophoretic Mobility Shift Assays (EMSA) for NF-κB

Whole cell extracts were prepared and analyzed for DNA binding activity using the HIV-LTR tandem κB oligonucleotide as κB probe (Jacque et al, 2005). Use of whole cell extracts to analyze NF-κB DNA-binding status by EMSA was previously validated by comparing nuclear and total NF-κB DNA-binding profiles and the observation of highly similar NF-κB DNA-binding activity, indicating that NF-κB binding emanates from nuclear proteins (Eluard et al, 2021).

### 7. RNA-sequencing and analysis

RNA sequencing librairies and sequencing were performed by the GenomeEast platform, a member of the 'France Génomique' consortium (Illkirch, France). Briefly, RNA-Seq libraries were generated from 600 ng of total RNA using TruSeq Stranded mRNA LT Sample Preparation Kit (Illumina, San Diego, CA), according to manufacturer's instructions (TruSeq Stranded mRNA Sample Preparation Guide - PN 15031047: Rev.E Oct 2013). The final cDNA libraries were checked for quality and quantified using capillary electrophoresis with 2100 Bioanalyzer (Agilent Technologies France, Les Ulis) and further sequenced on a HiSeq 4000 sequencer (Illumina) as single-end 50 base reads following Illumina's instructions.

Reads were preprocessed using cutadapt 1.10 (Martin, 2011) in order to remove adaptors and low-quality sequences and reads shorter than 40 bp were removed for further analysis. Remaining reads were mapped to Homo sapiens rRNA and ERCC Spike-In sequences using bowtie 2.2.8 (Langmead & Salzberg, 2012) and reads mapped to those sequences were removed for further analysis. Remaining reads were aligned to hg38 assembly of Homo sapiens with STAR 2.5.3a (Dobin et al, 2013). Gene quantification was performed with htseq-count 0.6.1p1 (Anders et al, 2015), using "union" mode and Ensembl 94 annotations. Differential gene expression analysis was performed using DESeq2 1.16.1 (Love et al, 2014). Bioconductor R package on previously obtained counts (with default options). P-values were adjusted for multiple testing using the Benjamini and Hochberg method (Benjamini 1995).

### 8. Quantitative real-time polymerase-chain reaction

Total RNA extraction and reverse transcription were performed as previously described (Jacque et al 2005). Real-time PCR analysis was carried out with Light Cycler FastStart DNA Masterplus SYBR Green I on a LightCycler 1.5 (Roche Applied Science). All values were normalized to the level of hypoxanthine-guanine phosphoribosyltransferase (HPRT) mRNA. Primers sequences are listed in table 7 below.

**Table 7**

| **Gene** | **Primer Sequences** |
|---|---|
| ABCG1 | F (SEQ ID NO:10): 5'-tcagggacctttcctattcg-3' |
| | R (SEQ ID NO:11): 5'- ttcctttcaggagggtcttgt-3' |
| ALDH8A1 | F (SEQ ID NO:12): 5'- aaccgtcaggtccagcttt-3' |
| | R (SEQ ID NO:13): 5'- cactatagatgctcttctggacaaa -3' |
| ALPI | F (SEQ ID NO:14): 5'-catggaccgcttcccata-3' |
| | R (SEQ ID NO:15): 5'- ggcacctgtctgtccacat-3' |
| CDS2 | F (SEQ ID NO:16): 5'- cgattttcccaggatgacag-3' |
| | R (SEQ ID NO:17): 5'- tctacctttgcttctgactctgac-3' |
| CYP1 B1 | F (SEQ ID NO:18): 5'- aacgtaccggccactatcac-3' |
| | R (SEQ ID NO:19): 5'- ctcgagtctgcacatcagga-3" |
| ELOVL4 | F (SEQ ID NO:20): 5'- catttggcccatggattc-3' |
| | R (SEQ ID NO:21): 5'-ccaatggtcacatggaattg-3' |
| PTGIS | F (SEQ ID NO:22): 5'- tgaaaaggccaggatgaaac-3' |
| | R (SEQ ID NO:23): 5'- ggaggttggtatacatggcttc-3' |
| PRKAR2B | F (SEQ ID NO:24): 5'- cccagagcagctacaatcact -3' |
| | R(SEQ ID NO:25): 5'- tttttggcattgtttttcaca-3' |
| UGT2B15 | F (SEQ ID NO:26): 5'- gaaaaagaaacagcgtgaaattg-3' |
| | R (SEQ ID NO:27): 5'- ttctccaatcctcttattttcctc-3' |
| HPRT | F (SEQ ID NO:28): 5'-ggcgtcgtgattagtgatg-3' |
| | R (SEQ ID NO:29): 5'-gcacacagagggctacaatgt-3' |

| | |
|---|---|
| F: Forward primer. R: reverse primer. | |

### 9. Metabolomics by NMR

### Sample preparation

Extracellular media, along with aqueous and lipophilic extracts from cell pellets were prepared following protocols as published elsewhere (Beckonert, O.; Keun, H. C.; Ebbels, T. M. D.; Bundy, J.; Holmes, E.; Lindon, J. C.; Nicholson, J. K. Nature Protocols 2007, 2, 2692.). For each cell line, 10 independent samples of extracellular medium and cell pellets were prepard and analyzed.

All NMR experiments were recorded on a 500 MHz Bruker Avance III spectrometer equiped with a dual ¹H / ¹³C cryogenically cooled probe head, and a SampleXPress changer operating at room temperature. The operating software was TopSpin 2.1.

### 1D 1H Spectra

The pulse sequence *noesygppr1d* from the Bruker library was used to acquire ¹H *1D NOESY-presat* spectra with water suppression. The rf power for the presaturation of the water signal was set to 4.275 mW. The mixing time was set to 100 ms. The two spoil gradients were applied with amplitudes G₁ = 32.85 G.cm⁻¹ and G₂ = -6.57 G.cm⁻¹ respectively. The duration of the p/2 pulse was 10.6 µs for ¹H. A free induction decay of 32k points (acquisition time: 1.6 s) was acquired, with 256 scans, 2 dummy scans, and a recycling delay of 2 s between scans. The spectral window was set to 20 ppm.

### Metabolites' identification

For each NMR spectrum, standard processing protocol was applied, including Fourier transform, phase correction, baseline correction and calibration. Spectra were aligned to a reference signal using trimethylsilylpropanoic acid (TSP) as a chemical shift standard (-0.016 ppm). For metabolite identification, ¹H NMR spectra were analyzed using Chenomx NMR Suite 8.0 (Chenomx Inc.). Metabolites identification was performed using the spectral reference library, and fitting peaks intensity and location to the observed signals.

### Statistical Analysis

The processing of the raw data was carried out using NMRProcflow, including baseline correction, chemical shift calibration, data alignment and variable sized bucketing (VSB) (Jacob et al 2017). For all datasets, a threshold corresponding to a signal to noise ratio of 3 was applied. SIMCA 15 (Umetrics)was used to perform principal component analysis (PCA) and orthogonal projections to latent structures discriminant analysis (OPLS-DA. Pareto scaling was used in both analyses. MetaboAnalyst 4.0 (Chong et al, 2018) was used to perform t-tests and determine p-values, and to generate box-plots.

### 10. Lipid content

### Intracellular lipid quantification by Oil red O staining

Cells were washed with phosphate buffered saline solution (PBS) and fixed with formalin 10 % and isopropanol 60 %. After two washes with H₂O, intracellular neutral lipids were stained with Oil Red O for 10 min at room temperature. Image acquisition was performed using optical microscopy (magnification: 20X) for at least 6 fields/well, and quantification of Oil Red O staining was made using image J software and expressed as lipid droplet area.

*Total triglyceride content*Lipids were extracted using the Folch method using chloroform/methanol (2:1, v/v). Triglyceride content was quantified using a colorimetric diagnostic triglyceride assay kit (Triglycerides FS; Diasys), and further normalized to whole cell extract protein concentration as measured by BCA Protein Assay kit (23227, Pierce).

### 11. Fatty acid metabolic flux

### [1-¹⁴C]Oleic acid oxidation rate

[1-¹⁴C]Oleic acid was obtained from GE Healthcare. Huh7 cells were incubated in serum-free media in the presence of [1-¹⁴C]Oleic acid for 3 hours. At the end of the incubation, cell culture medium was collected to evaluate [¹⁴C]C0₂ production in the presence of benzothenium hydroxide, and [¹⁴C] ASMs (acid-soluble metabolites that correspond to TCA cycle intermediates) production using 40% perchloric acid. [¹⁴C]C0₂ production was directly quantified after addition of scintillation liquid to the benzothenium hydroxide mixture. [¹⁴C]ASMs were isolated following of the centrifugation of the perchloric acid-medium mixture, and collection of the supernatant. [¹⁴C]ASMs were quantified after addition of scintillation liquid using a scintillation counter (Packard). FA oxidation rate were calculated from [¹⁴C]C0₂ and [¹⁴C] ASMs and expressed as nmoles of FA oxidized over 3 hours per mg of proteins. Protein content was determined using the BCA protein assay (Thermo Fisher Scientific).

### [1-¹⁴C]Oleic acid esterification into lipid fraction and TG. PL, DAG quantification

Esterification rate of [1-¹⁴C]Oleic acid] was measured in cells by tracing ¹⁴C incorporation in lipidnewly synthetized lipids. Cells were treated with [1-¹⁴C]Oleic acid as above and cellular lipids were extracted using the Folch method. The lipid extract was re-dissolved in chloroform/methanol (2:1, v/v), and separated on thin-layer silicate chromatography (Merck) using petroleum ether-diethyl ether-acetic acid (85:15:0.5, v/v/v) as the mobile phase. Triacylglycerols (TG), DAG (Di-acylglycerols) and PL (Phospholipids) were identified using standard oils and coloration with iodine vapor. Specific bands for each lipid subclass were cut off and placed in scintillation liquid. [¹⁴C]TG, [¹⁴C]DAG and [¹⁴C]PL and [¹⁴C]FA products were quantified after addition of scintillation liquid using a scintillation counter (Packard). FA esterification rates were expressed in nmoles of esterified FA over 3 hours per mg of proteins. Protein content was determined using the BCA protein assay (Thermo Fisher Scientific).

### 12. Statistical analysis

Kaplan-Meier estimate of overall survival of the TCGA cohort (HCC patients, n=364) according to human RelB mRNA expression (RNAseq), was performed using online KMplotter software and data available at <kmplot.com>. P value was determined by Log-rank test (Mantel-Cox, Menyhart et al, 2018).

Statistical significance for all other parameters was assessed using unpaired t-test (Prism 8.0, GraphPad Software). A value of p=0.05 was considered as statistically significant with the following degrees: * p<0.05; **p<0.01; ***p<0.001; ****p<0.0001.

### RESULTS

### A. RELB AFFECTS HUH7 CANCER CELL METABOLISM THROUGH DEREGULATION OF FATTY ACID AND LIPID PATHWAYS

### 1. Identification of a RelB gene expression signature associated with fatty acid and lipid metabolism

Endogeneous RelB DNA binding activity was detected in the parental Huh7 by EMSA shift assay, thereby signing endogeneous RelB activity in this HCC cell line (not shown). Moreover, high RelB activity upon RelB overexpression by stable lentiviral transduction was validated by immunoblotting and EMSA shift assay (not shown). In addition, RelB expression knockdown by lentiviral transduction of a shRNA targeting RelB in Huh7 cells strongly decreases RelB expression as compared to a shRNA control as evaluated by immunoblotting experiments (not shown).

A specific transcriptomic signature linked to lipid metabolism controlled by RelB in these HCC cells was identified by using these two different sets of genetically engineered Huh7 cell lines: (i) the shRNA inhibition model of RelB expression knock-down, and (ii) the RelB cDNA overexpression model as described above. RNA-seq transcriptomics allows to identify a ReIB-dependent transcriptomic signature of 28 genes implicated in lipid metabolism, either directly or indirectly (in either transport, detoxification, synthesis, oxidation or storage of lipids, Table 8).

**Table 8**

| **Ensembl gene ID** | **Gene name** | **Expression level (ReIB inhibition)** | | **Expression level (RELB overexpression)** | |
|---|---|---|---|---|---|
| | | **control** | **shRelB** | **control** | **WT RelB cDNA** |
| ENSG00000169429 | **CXCL8** | 0.7981 | 1.462 | 0.3214 | -2.581 |
| ENSG00000120937 | **NPPB** | 0.612 | 1.288 | -0.4216 | -1.478 |
| ENSG00000156735 | **BAG4** | -0.2229 | 0.3698 | 0.4336 | -0.5805 |
| ENSG00000242689 | **CNTF** | -0.4728 | 0.6371 | 0.339 | -0.5033 |
| ENSG00000100003 | **SEC14L2** | -0.03717 | -1.03 | -0.2853 | 1.353 |
| ENSG00000197971 | **MBP** | -0.06002 | -0.7927 | -0.4174 | 1.27 |
| ENSG00000160179 | **ABCG1** | -0.3513 | -1.211 | -0.579 | 2.141 |
| ENSG00000132470 | **ITGB4** | -0.1164 | -0.711 | -0.1955 | 1.023 |
| ENSG00000182600 | **SNORC** | -0.5382 | -1.241 | -0.06896 | 1.848 |
| ENSG00000157765 | **SLC34A2** | -1.064 | -1.817 | -0.39 | 3.271 |
| ENSG00000124813 | **RUNX2** | -0.06596 | -0.9825 | 0.1125 | 0.936 |
| ENSG00000140398 | **NEIL1** | -0.7439 | -1.385 | -0.2805 | 2.41 |
| ENSG00000118402 | **ELOVL4** | -0.5545 | -1.225 | -0.2288 | 2.008 |
| ENSG00000101290 | **CDS2** | -0.03444 | -0.7043 | -0.007579 | 0.7463 |
| ENSG00000198099 | **ADH4** | -0.1895 | -1.17 | -0.02081 | 1.381 |
| ENSG00000118514 | **ALDH8A1** | -0.3254 | -0.9184 | 0.1641 | 1.08 |
| ENSG00000196620 | **UGT2B15** | 0.7545 | -0.002148 | -2.138 | 1.386 |
| ENSG00000109424 | **UCP1** | 0.08658 | -0.6076 | -0.8505 | 1.371 |
| ENSG00000124212 | **PTGIS** | 0.03204 | -0.7399 | -1.474 | 2.182 |
| ENSG00000100311 | **PDGFB** | 0.375 | -0.2777 | -0.7288 | 0.6315 |
| ENSG00000005884 | **ITGA3** | 1.035 | 0.2369 | -2.69 | 1.418 |
| ENSG00000173612 | **GPRC6A** | 0.2676 | -0.327 | -0.795 | 0.8545 |
| ENSG00000163295 | **ALPI** | 0.9273 | 0.06816 | -2.175 | 1.179 |
| ENSG00000138061 | **CYP1B1** | 0.2881 | -0.4553 | -0.7459 | 0.913 |
| ENSG00000005249 | **PRKAR2B** | 1.187 | 0.4206 | -1.802 | 0.194 |
| ENSG00000131370 | **SH3BP5** | 0.5039 | -0.1673 | -0.8317 | 0.4951 |
| ENSG00000171791 | **BCL2** | 0.9038 | 0.06822 | -1.859 | 0.8872 |
| ENSG00000168918 | **INPP5D** | 0.5009 | -0.1857 | -0.5406 | 0.2253 |

The impact of WT RelB on the mRNA expression levels of several of these genes implicated in essential processes of lipids metabolism as transport, detoxification, synthesis, oxidation and storage was further validated by RT-PCR in the WT RelB overexpression Huh7 model (not shown).

### 2. RelB decreases intracellular lipid content and promotes fatty acid metabolism

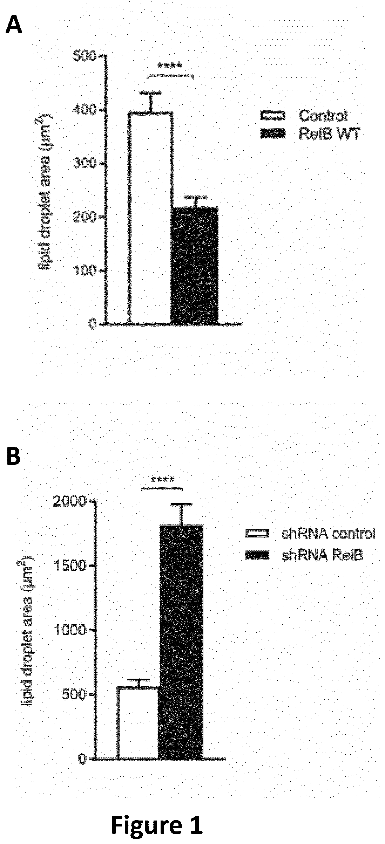

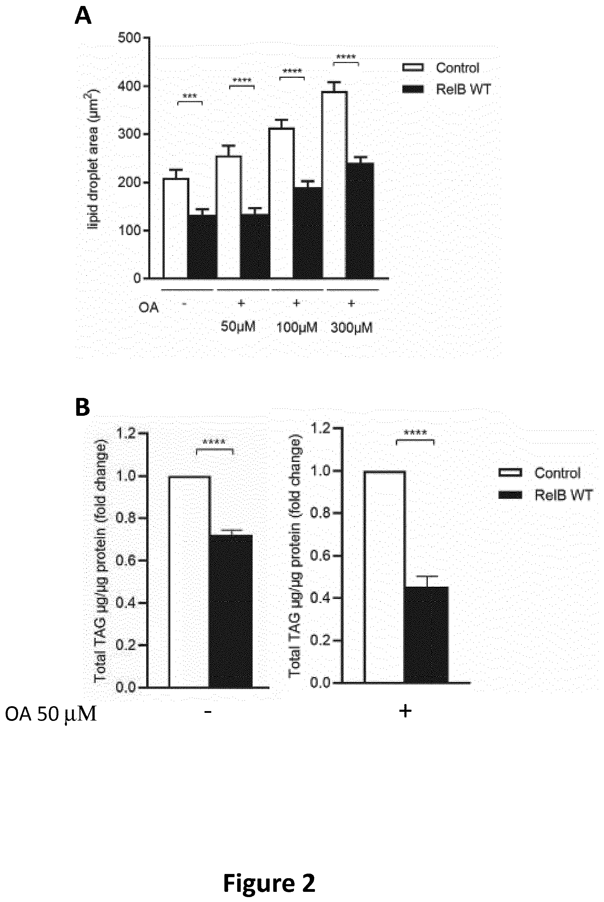

To go one step further, we then evaluated how RelB impacts on lipid storage within lipid droplets by Oil red O staining. High level of RelB expression strongly decrease TG storage in lipid droplets (Figure 1A), whereas RelB expression knockdown massively increases it (Figure 1B). The strong impact of RelB in lowering lipid storage was also seen in response to exogenous supplied oleate (Figure 2A) and palmitate (not shown). Accordingly, high levels of RelB expression induced a strong decrease of total intracellular TG content in the absence or presence of exogenous oleate (Figure 2B).

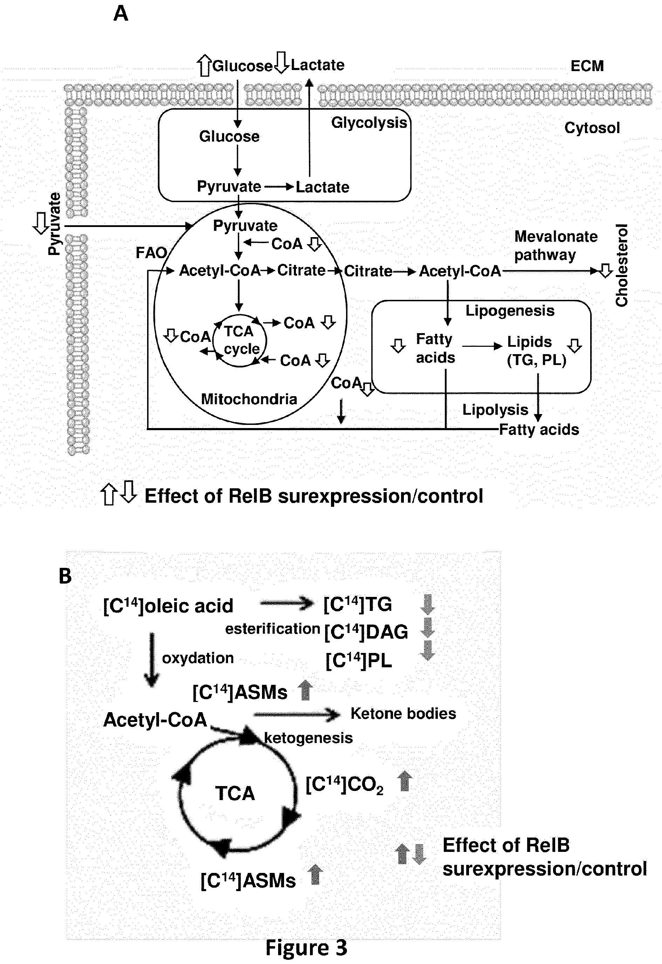

The massive decrease of intracellular lipids was further confirmed by metabolomics by NMR (organic phase) (Figure 3A and Table 9). Metabolomics by NMR also revealed a ReIB-dependent increase of intracellular choline and choline-derived lipids (acetyl-choline and phosphatidyl choline), thus indicative of Increased choline metabolism. Abnormal choline-metabolism is well reported as a metabolic hallmark linked to pro-tumor lipid remodeling. Interestingly, NMR analysis of extracellular medium reveal an increase of glucose levels along with a decrease of lactate levels in WT RelB overexpressing Huh7 cells as compared to the control cell line (Figure 3A), thus suggesting a parallel decrease in glycolysis. In line, a decrease of the extracellular acidification rate (ECAR), as measured on a Seahorse analyzer, was observed in WT RelB Huh7 cells (not shown). Moreover, a decrease in extracellular pyruvate levels upon RelB overexpression is observed, thereby indicative of an increase of its consumption to feed the TCA cycle through its conversion to Acetyl-coA. In line, a decrease of intracellular Coenzyme A (CoA) was observed in WT RelB Huh7 cells, a metabolites implicated in several metabolic steps such as pyruvate conversion to Acetyl-CoA, and Fatty Acid oxidation (FAO) leading to the conversion of fatty acids (FA) to Acetyl-CoA to feed the TCA cycle. Altogether, these data indicate that RelB controls lipid and FA metabolism in Huh7 HCC cells, leading to a metabolic rewiring towards mitochondrial oxidative phosphorylation.

Table 9 below summarizes the ReIB-dependent metabolomic signature cells related to lipid and FA metabolism.

**Table 9**

| **Insoluble fraction** | |
|---|---|
| **Linoleic acid** | decrease |
| **Phospholipids** | decrease |
| **Triglycerides** | decrease |
| **Monounsaturated fatty acids (MUFAs)** | decrease |
| **Unsaturated fatty acids UFA** | decrease |
| **Fatty acids other than UFAs and MUFAs** | decrease |
| **Cholesterol** | decrease |

| **Soluble fraction** | |
|---|---|
| **Choline** | increase |
| **Ace-choline** | increase |
| **O-P choline** | increase |

### 3. RelB reprogram fatty acid metabolism towards fatty acid oxidation and oxidative energetic metabolism

Figure 3B summarizes the results related to impact of RelB on the [1-¹⁴C]-oleic acid metabolic flux analysis (ReIB overexpressing Huh7 cells).

To directly assessed how RelB impacts on fatty acid metabolism flows (i.e. the balance between FA esterification vs FA oxidation), we performed an oleate metabolic flux analysis using [1-¹⁴C]Oleic acid.

[1-¹⁴C]-oleate can be either esterified into lipids, such as diacylglycerols (DAG), phospholipids (PL) and triacylglycerols (TG), or oxidized to feed the TCA cycle and produce ketone bodies. Fatty acid oxidation can either be complete when FA are oxidized into CO₂ or incomplete when FA are incorporated into ASMs, including shortcut acylcarnitines, TCA intermediates and acetyl-CoA (Figure 3B). Remarkably, RelB strongly decreased the rate of [1-C14]Oleic acid esterification, as evidenced by a lower [1-¹⁴C]-oleic acid esterification rate into [¹⁴C]PL, [¹⁴C]DAG, and [¹⁴C] TG (Figure 3B). Moreover, increased RelB expression levels in Huh7 cells significantly increased the oleate oxidation rate (Figure 3B). Altogether, these data indicate that increased RelB activity promotes causes a metabolic reprograming toward fatty acid oxidation at the cost of fatty acid esterification, and thus lowering total intracellular lipid content.

In addition, we also observed that the alternative RelB NF-κB subunit impacts on Huh7 cell energy homeostasis by enhancing oxygen consumption rate (OCR) as measured by Seahorse assay (not shown) as well as mitochondrial ATP content (not shown) upon exogenous supplied oleate, thus indicating that RelB promotes oxidative phosphorylation. Collectively, our data uncovered that RelB reprograms HCC cell towards FAO and subsequently oxidative phosphorylation.

### 4. RelB promotes cell growth and survival through fatty acid oxidation

Metabolic reprograming is recognized as a hallmark of cancer giving selective benefit for cancer cell growth and survival.

Endogenous RelB expression knockdown induced a time-dependent induction of Huh7 cell death (Figure 4A), reaching 80% at 96 hours post-transduction with the shRNA targeting ReIB, while almost no cell death was observed in the shRNA control Huh7 cells. RelB overexpression induced a significant increase of Huh7 cell growth (Figure 4B). Most importantly, in presence of 4-bromocrotonic acid (4-BCA), an inhibitor of fatty acid oxidation (Grünig et al, 2018), the ReIB-dependent increase in Huh7 cell number was lost and cell growth rate was even lowered compared to the control cells (Figure 4B).

Since RelB controls FAO, and thereby mitochondrial oxidative phosphorylation, we next assessed the contribution of RelB in Huh7 survival upon treatment with lipotoxic amount of palmitic acid (Figure 4C). RelB overexpression drives resistance to palmitate induced Huh7 cell death. Most remarkably, RelB pro-survival effect was almost completely abolished in 4-BCA). Altogether, our data indicate that RelB gives a selective benefit to Huh7 HCC cell growth and protects liver cancer cells from cellular stress through the rewiring of energetic metabolism towards FAO.

### 5. High RelB expression is associated with worse overall survival in HCC patients.

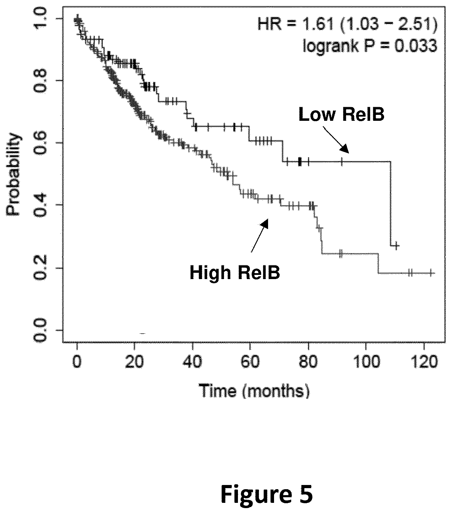

In a next step, it was important to assess how RelB activation affected HCC patient outcome. Remarkably, an analysis of RNA seq data available at Kaplan-Meier Plotter (<https://kmplotcom>, Figure 5) shows that RelB mRNA expression levels was significantly associated with worse overall survival of HCC patients (n=364, P=0,033). Further, it has been found that the RelB dependent 28 genes signature related to lipid metabolism of the is associated with worse overall survival of HCC patients and is correlated with RelB mRNA expression levels (not shown).

In conclusion, these results show that RelB 1) controls genes implicated in metabolism and associated to lipids, 2) decreases lipid content and storage, 3) induces metabolic reprograming towards fatty acid oxidation, and thereby oxidative phosphorylation, at the cost of lipid synthesis and 4) uses fatty acid oxidation as an essential nutriment for cancer cell growth and survival. More particularly, this RelB dependent reprograming provides a selective advantage for cell growth and survival in Huh7 hepatocarcinoma cell line. RelB is also found to be associated with a worse prognostic in HCC patients.

Considering the phenotype of Huh7 shRelB cells (inhibition model), results show that targeting RelB is of outmost interest for therapeutic intervention and development of treatments of these cancers.

Also, cancers showing an activated ReIB, are susceptible to exhibit a metabolite reprograming towards lipids metabolism as described above, which implies reconsidering therapeutic intervention in these cancers.

### B. LIPID METABOLISM IS A THERAPEUTIC TARGET IN RELB POSITIVE LIQUID CANCER CELL

RelB NF-κB subunit is frequently activated in DLBCL patients and cell lines, independently of their ABC or GCB subtypes. RelB activity predicted worse overall survival upon immunochemotherapy. RelB was found to promoting DLBCL cell survival upon treatment with genotoxic agents (Eluard et al, 2021). Whether RelB activation might be connected to the OxPhos metabolic status of DLBCL cells and to the metabolic reprograming is analyzed below.

### 1. The RelB positive-MD901 DLBCL cell line is an OxPhos-DLBCL

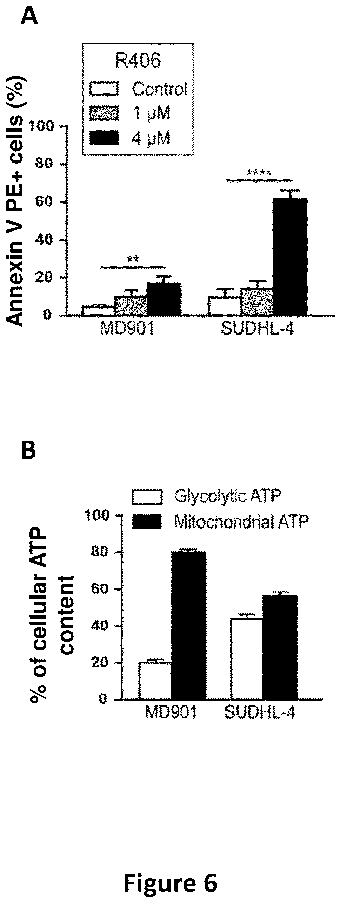

The ABC DLBCL cell line MD901 has been previously described as exhibiting a strong constitutive RelB DNA-binding activity (Eluard et al, 2021). Whether this DLBCL cell line is an OxPhos or a BCR-DLBCL cell line was tested by assaying its sensitivity to R406, an ATP competitive inhibitor of the spleen tyrosine kinase (SYK) that only induces apoptosis in DLBCL cell lines having an active BCR signaling, whereas OxPhos-DLBCLs that do not display functional BCR signaling are known to be insensitive to this inhibitor (Chen et al, 2008). As presented in Figure 6A, MD901 DLBCL cell line is insensitive to R406-induced apoptosis, in contrast to the BCR-DLBCL cell line SUDHL-4 (Chen et al, 2008). BCR-dependent DLBCL cell lines are known to rely more on a glycolytic metabolism (Norberg et al, 2017). This is confirmed by data presented in Figure 6B wherein MD901 DLBCL cells are shown to produce only a low level of glycolytic ATP, on the contrary to what was seen with the reported glycolytic BCR SUDHL-4 DLBCL cell line (Figure 6B). The MD901 DLBCL cell line is therefore a new OxPhos-DLBCL cell line.

### 2. RelB NF-κB subunit controls energy homeostasis in liquid cancer cell showing activation of ReIB.

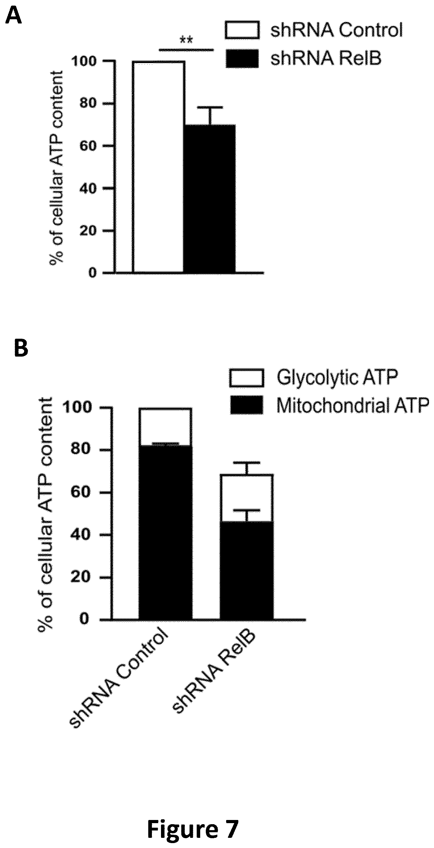

To assess the possible role of RelB in the mitochondrial metabolism of MD901 DLBCL cells, a stable RelB knockdown approach by stable RNA interference was used (using either a lentivirus carrying an shRNA targeting RelB (shRNA ReIB) or a scrambled control (shRNA control)). RelB protein levels are found significantly inhibited upon RelB knockdown (not shown). Importantly, knockdown of RelB expression also results in a marked decrease in constitutive RelB binding activity without affecting RelA binding activity (not shown). RelB inhibition markedly reduced total ATP levels under normal culture conditions (Figure 7A) without affecting the glycolytic ATP in MD901 DLBCL cells (Figure 7B). RelB is therefore critical for DLBCL cell energy production by directing metabolism towards mitochondrial ATP production.

### 3. RelB protects DLBCL cells from mitochondrial stress-induced apoptosis

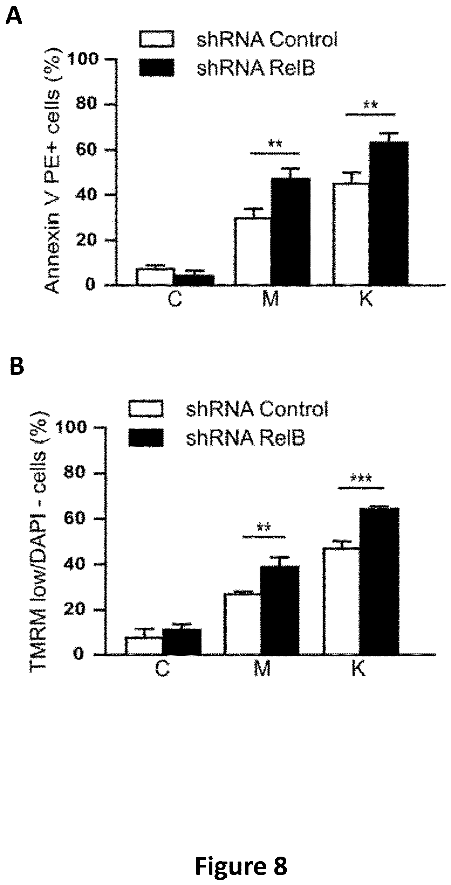

Metformin, or L-asparaginase (Kidrolase^{®}), are two antimetabolic FDA approved drugs targeting the mitochondrial metabolism. Metformin is a widely prescribed antihyperglycemic biguanide drug to type II diabetics, inhibiting specifically complex I of the mitochondrial electron transport chain (Inzucchi et al, 1998). L-asparaginase (Kidrolase^{®}) catalyzes the hydrolysis of asparagine and glutamine into aspartate and glutamate, respectively, accompanied by mitochondria dysfunction (Parmentier et al 2015 ; Takahashi et al, 2017). Metformin and Kidrolase^{®} both induced significant apoptosis in MD901 DLBCL cell line as evaluated by Annexin V/DAPI staining (Figure 8A). Further, ReIB-expression knockdown markedly increased either Metformin or Kidrolase^{®} induced apoptosis. (Figure 8A). This effect is found associated with an increase in loss of mitochondrial potential (ΔΨm) (Figure 8B), and cleavage of caspase 3 (not shown). Further, knockdown of RelB expression is found to lead to an even more pronounced cell death induced by metformin. Therefore, RelB inhibition enhances the pro-apoptotic efficacy of anti-metabolic drugs targeting mitochondrial metabolism, and correlatively, RelB inhibition is found to render DLBCL cells more susceptible to mitochondrial stress, and to play a role in metabolism governance of these cancer cells.

### 4. Inhibition of fatty acid oxidation induces cell death in RelB positive liquid cancer cells

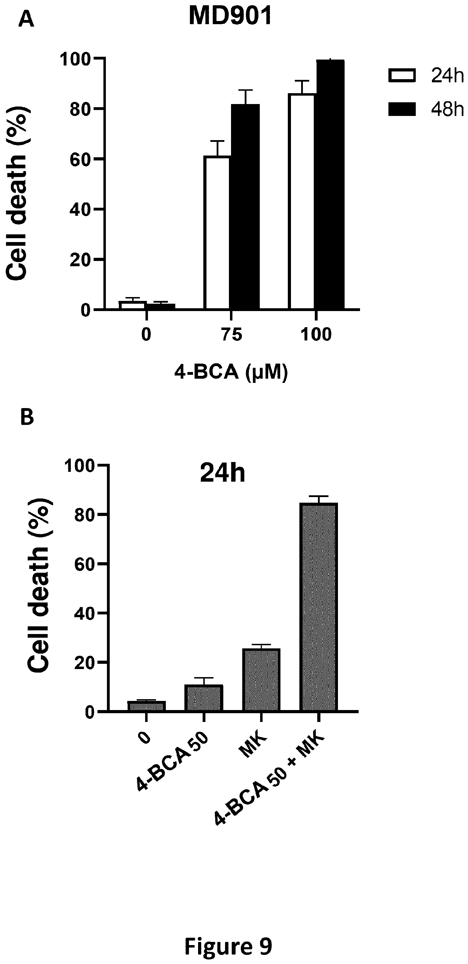

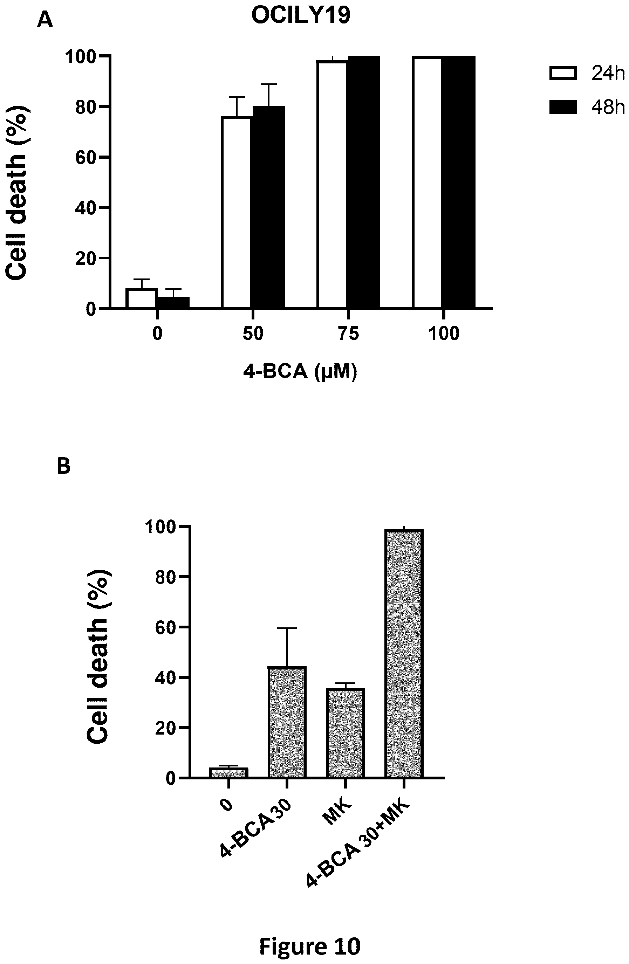

Inhibition of fatty acid oxidation (4-BCA treatment) in OxPhos DLBCL cell lines -e ither MD901 (Figure 9A) or Oci-Ly19 (Figure 10A), induces a massive and rapid cell death, as soon as 24 hours post-treatment with 4-BCA. In comparison, glycolytic BCR SUDHL-4 cells are much less sensitive to 4-BCA treatment (not shown). Moreover, as shown in Figures 9B and 10B, exposure of DLBCL cells to lower doses of 4-BCA as used above, in combination with Metformin and Kidrolase (2 FDA approved inhibitors of mitochondrial metabolism) resulted in at least the same level of cell death, as soon as 24 hours (Figure 9B & Figure 10B).

### 5. Inhibition of fatty acid synthesis induces cell death in RelB positive liquid cancer cells

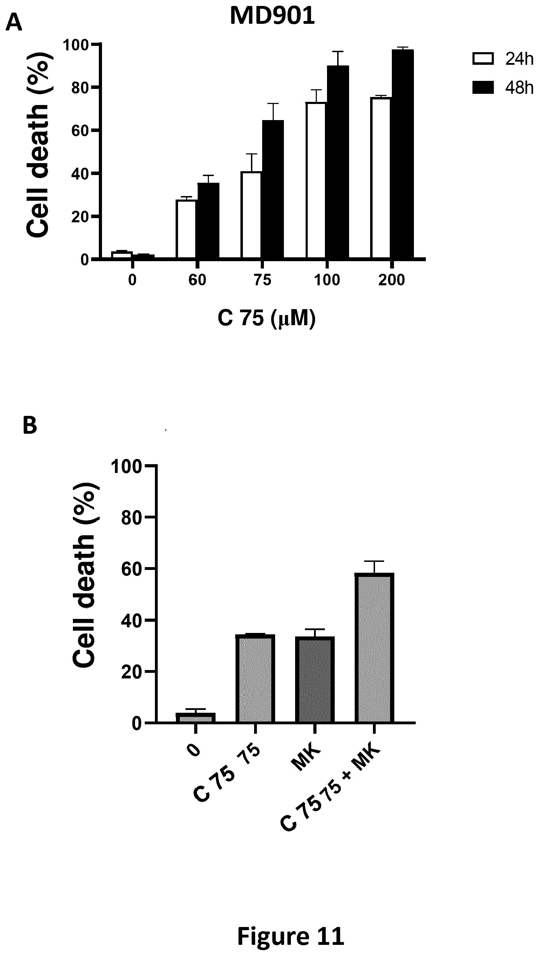

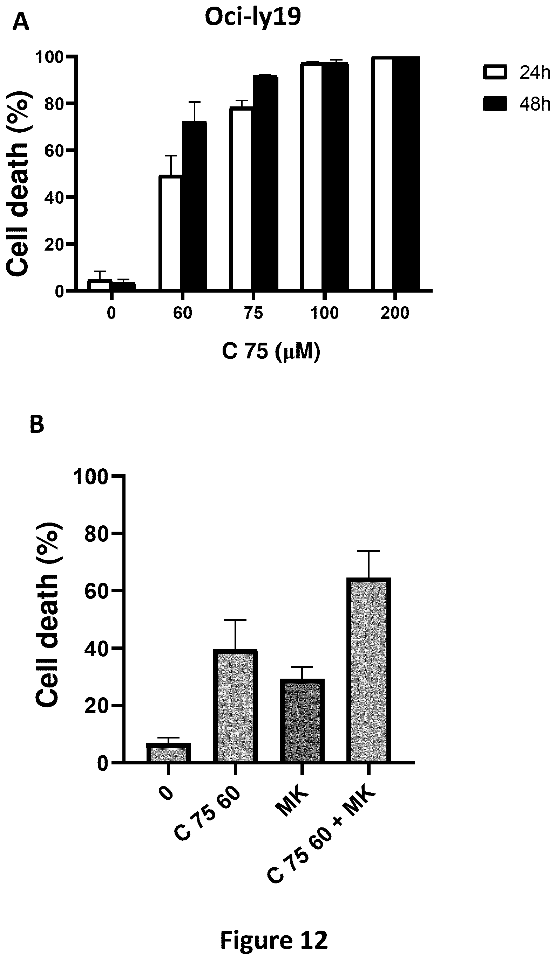

Inhibition of fatty acid synthesis (C75 treatment, a fatty acid synthase (FAS) inhibitor) in OxPhos DLBCL cell lines - either MD901 (Figure 11 A) or Oci-Ly19 (Figure 12A), induces massive and rapid cell death, as soon as 24 hours post-treatment with C75. In comparison, glycolytic BCR SUDHL-4 cells are much less sensitive to C75.

Moreover, as shown in Figures 11B and 12B, exposure of DLBCL cells to C75 combined with metformin and Kidrolase^{®} resulted in at least the same level of cell death, as soon as 24h (Fig.11B, Fig.12B).

Collectively, these results show that RelB plays a crucial role in the maintenance of DLBCL cell energy metabolism and is a crucial positive regulator of DLBCL cell survival upon mitochondrial stress. Further, RelB constitutive activation as in DLBCL MD901 cells induces a metabolic reprograming towards oxidative energy metabolism, thus confirming the role of RelB in energetic metabolism, and its implication in cell metabolic reprograming as shown by the higher sensitivity of RelB activated MD901 or Oci-Ly19 DLBCL cells to lipid metabolism (both Fatty Acid Oxidation and lipid synthesis) inhibition.

### C. CONCLUSION

RelB is found to govern cell metabolism reprograming towards oxidative energy metabolism and more particularly the use of fatty acid oxidation and mitochondrial respiration of cancer cells with an activated RelB of either solid or liquid cancers. Metabolism reprograming confers a selective advantage for tumor cell growth and is associated with refractory cancers. RelB constitutes therefore a new prognostic marker for metabolism reprograming in cancer cells and a valuable tool in management of cancer treatment. Also, accordingly, RelB inhibitors and/or lipid metabolism inhibitors in RelB activated cancers provide valuable therapeutic targets, optionally in combination with antimetabolic drugs, when available.

### REFERENCES

Anders S., Pyl P., and Huber W., "HTSeq-a python framework to work with high-throughput sequencing data," Bioinformatics, vol. 31, no. 2, pp. 166-169, 2015. Ausubel et al. CURRENT PROTOCOLS IN MOLECULAR BIOLOGY, 1989.
Authier H, Billot K, Derudder E, et al. IKK phosphorylates RelB to modulate its promoter specificity and promote fibroblast migration downstream of TNF receptors. Proc Natl Acad Sci USA. 2014;111(41):14794-14799.
Baud V, Collares D. Post-Translational Modifications of RelB NF-κB Subunit and Associated Functions. Cells. 2016;5(2):22.
Benjamini Y. and Hochberg Y., "Controlling the false discovery rate: A practical and powerful approach to multiple testing," J R Statist Soc B, vol. 57, no. 1, pp. 289-300, 1995.
Love M., Huber W., and Anders S., "Moderated estimation of fold change and dispersion for RNA-seq data with DESeq2," Genome Biol, vol. 15, no. 12, p. 550, 2014.
Chen L, Monti S, Juszczynski P, Daley J, Chen W, Witzig TE, et al. SYK-dependent tonic B-cell receptor signaling is a rational treatment target in diffuse large B-cell lymphoma. Blood. 15 févr 2008;111(4):2230-7.
Chong J, Soufan O, Li C, Caraus I, Li S, Bourque G, Wishart DS, Xia J. MetaboAnalyst 4.0: towards more transparent and integrative metabolomics analysis. Nucleic Acids Res. 2018 Jul 2;46(W1):W486-W494.
Currie, E., Schulze, A., Zechner, R., Walther, T. C. & Farese, R. V. Cellular Fatty Acid Metabolism and Cancer. Cell Metabolism 18, 153-161 (2013).
Dang, C. V. Links between metabolism and cancer. Genes & Development 26, 877-890 (2012).
DeBerardinis, R. J. & Chandel, N. S. Fundamentals of cancer metabolism. Sci. Adv. 2, e1600200 (2016).
Ding, Z.-B. et al. Autophagy activation in hepatocellular carcinoma contributes to the tolerance of oxaliplatin via reactive oxygen species modulation. Clin Cancer Res 17, 6229-6238 (2011).
Dobin A, Davis C, et al. "STAR: ultrafast universal RNA-seq aligner," Bioinformatics, vol. 29, no. 1, pp. 15-21, 2013.
Eluard B, Nuan-Aliman S, Faumont N, Collares D, Bordereaux D, Montagne A, et al . The alternative RelB NF-κB subunit is a novel critical player in diffuse large B-cell lymphoma. Blood. 2022 Jan 20;139(3):384-398.
Geiss GK, et al. Direct multiplexed measurement of gene expression with color-coded probe pairs. Nat Biotechnol. 2008 Mar;26(3):317-25.
Grünig, D., Duthaler, U. & Krähenbühl, S. Effect of Toxicants on Fatty Acid Metabolism in HepG2 Cells. Front Pharmacol 9, 257 (2018).
Hanahan, D. & Weinberg, R. A. Hallmarks of Cancer: The Next Generation. Cell 144, 646-674 (2011).
Hofmarcher T, Brådvik G, Svedman C, Lindgren P, Jönsson B and Wilking N. Comparator Report on Cancer in Europe 2019 - Disease Burden, Costs and Access to Medicines. IHE Report 2019:7, IHE: Lund, Sweden.
Huang, Y. et al. Serum starvation-induces down-regulation of Bcl-2/Bax confers apoptosis in tongue coating-related cells in vitro. Mol Med Rep 17, 5057-5064 (2018).
Inzucchi SE, Maggs DG, Spollett GR, Page SL, Rife FS, Walton V, et al. Efficacy and metabolic effects of metformin and troglitazone in type II diabetes mellitus. N Engl J Med. 26 mars 1998;338(13):867-72
Jacob, D., Deborde, C., Lefebvre, M., Maucourt, M. and Moing, A. (2017) NMRProcFlow: A graphical and interactive tool dedicated to 1D spectra processing for NMR-based metabolomics, Metabolomics 13:36
Jacque E, Tchenio T, Piton G, Romeo P-H, Baud V. RelA repression of RelB activity induces selective gene activation downstream of TNF receptors. Proc Natl Acad Sci. 11 oct 2005;102(41):14635-40.
Kieusseian A, Chagraoui J, Kerdudo C, Mangeot P-E, Gage PJ, Navarro N, et al. Expression of Pitx2 in stromal cells is required for normal hematopoiesis. Blood. 15 janv 2006;107(2):492-500.
Köhler G, Milstein C. Derivation of specific antibody-producing tissue culture and tumor lines by cell fusion. Eur J Immunol. 1976;6(7):511-519.
Köhler G, Milstein C. Continuous cultures of fused cells secreting antibody of predefined specificity. Nature. 1975;256(5517):495-497.
Langmead Band S. Salzberg S, "Fast gapped-read alignment with Bowtie 2," Nat Methods, vol. 9, no. 4, pp. 357-359, 2012.
Maier HJ, Marienfeld R, Wirth T, Baumann B (2003) Critical role of RelB serine 368 for dimerization and p100 stabilization. J Biol Chem 278: 39242-392500eckinghaus A, Ghosh S. The NF-kappaB family of transcription factors and its regulation. Cold Spring Harb Perspect Biol. 2009;1 (4):a000034.
Marienfeld R, May MJ, Berberich I, Serfling E, Ghosh S, et al. (2003) RelB forms transcriptionally inactive complexes with ReIA/p65. J Biol Chem 278: 19852-19860.
Martin M., "Cutadapt removes adapter sequences from high-throughput sequencing reads," EMB- net.journal, vol. 17, no. 1, pp. 10-12, 2011.
Menyhart O, Nagy A, Gyorffy B. Determining consistent prognostic biomarkers of overall survival and vascular invasion in hepatocellular carcinoma. R Soc Open Sci., 2018 Dec 5;5(122):181006.
NeedlemanSB and Wunsch CD. A general method applicable to the search for similarities in the amino acid sequence of two proteins J. Mol. Biol. 1970 Mar;48(3):443-53.
Norberg E, Lako A, Chen P-H, Stanley IA, Zhou F, Ficarro SB, et al. Differential contribution of the mitochondrial translation pathway to the survival of diffuse large B-cell lymphoma subsets. Cell Death Differ. 2017;24(2):251-62.
Parmentier JH, Maggi M, Tarasco E, Scotti C, Avramis VI, Mittelman SD. Glutaminase activity determines cytotoxicity of L-asparaginases on most leukemia cell lines. Leuk Res. juill 2015;39(7):757-62.
Schouten JP, McElgunn CJ, Waaijer R, Zwijnenburg D, Diepvens F, Pals G (2002). "Relative quantification of 40 nucleic acid sequences by multiplex ligation-dependent probe amplification". Nucleic Acids Res. 30 (12): 57e-57.
Takahashi H, Inoue J, Sakaguchi K, Takagi M, Mizutani S, Inazawa J. Autophagy is required for cell survival under L-asparaginase-induced metabolic stress in acute lymphoblastic leukemia cells. Oncogene. juill 2017;36(30):4267-76.
Wang Z, Gerstein M, Snyder M. RNA-Seq: a revolutionary tool for transcriptomics. Nat Rev Genet. 2009 Jan;10(1):57-63. doi: 10.1038/nrg2484. Review.
Warburg, O. On the Origin of Cancer Cells. Science 123, 309-314 (1956).
Ward, P. S. & Thompson, C. B. Metabolic Reprogramming: A Cancer Hallmark Even Warburg Did Not Anticipate. Cancer Cell 21, 297-308 (2012).
Yoshida GJ. Metabolic reprogramming: the emerging concept and associated therapeutic strategies. J Exp Clin Cancer Res. 2015 Oct 6;34:111.

## Claims

1. An *in vitro* method for classifying a subject afflicted with a cancer as suffering from a cancer with a, or at risk of, metabolic reprograming towards fatty acids oxidation comprising a step of assaying the activation of RelB in a cancer cell sample from said cancer, wherein detecting said activation is indicative of, or of the risk of, the metabolic reprograming of said cancer towards fatty acids oxidation.

2. The *in vitro* method according to claim 1, further comprising a step of detecting an alteration in the expression of at least one gene selected from : CXCL8, NPPB, BAG4, CNTF, SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D.

3. The *in vitro* method according to any one of claim 1 or 2, wherein said cancer is a solid cancer selected from breast, prostate, pancreatic cancers, glioma, glioblastoma, ovarian, endometrial, skin or liver cancer, or a liquid cancer selected from a Hodgkin lymphoma, a non-Hodgkin lymphoma such as diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL) or leukemia.

4. The *in vitro* method according to any one of claims 1 or 2 wherein said cancer is hepatic cancer, more preferably hepatocellular carcinoma.

5. The *in vitro* method according to any one of claims 1 or 2 wherein said cancer is a non-Hodgkin lymphoma, more preferably a DLBCL.

6. An inhibitor of the expression of RelB and/or of the activity of RelB for use in inhibiting or preventing metabolic reprograming towards fatty acids oxidation in a cancer cell.

7. The inhibitor for use according to claim 6 wherein cancer cell shows a metabolic reprograming towards fatty acids oxidation.

8. The inhibitor for use according to claim 7 wherein said cancer cell shows an alteration in the expression of at least one gene selected from RELB, CXCL8, NPPB, BAG4, CNTF, SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D.

9. The inhibitor for use according to any one of claims 6 to 8, wherein the inhibitor of RelB expression and / or of the activity of RelB expression comprises at least:
- an antisense nucleotide, or a siRNA, or a shRNA, designed against RelB gene, and/or
- an anti-RelB antibody, a fragment or a derivative thereof.

10. The inhibitor for use according to any one of claims 6 to 9 wherein said cancer is a solid cancer selected from breast cancer, prostate cancer, pancreatic cancer, glioma, glioblastoma, ovarian, endometrial cancer, skin cancer, liver cancer, or a liquid cancer selected from a Hodgkin lymphoma, a non-Hodgkin lymphoma such as a diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL) or leukemia.

11. An inhibitor of lipid metabolism for use in treating a cancer showing an activated RelB.

12. The inhibitor of lipid metabolism for use according to claim 11, wherein said in said cancer, cancer cell further shows an alteration in the expression of at least one gene selected from CXCL8, NPPB, BAG4, CNTF, SEC14L2, MBP, ABCG1, ITGB4, SNORC, SLC34A2, RUNX2, NEIL1, ELOVL4, CDS2, ADH4, ALDH8A1, UGT2B15, UCP1, PTGIS, PDGFB, ITGA3, GPRC6A, ALPI, CYP1B1, PRKAR2B, SH3BP5, BCL2 and INPP5D.

13. The inhibitor of lipid metabolism for use according to claim 11 or 12, said inhibitor being selected from at least one compound of the group of soraphen-A, TOFA (5-(tetradecyloxy)-2-furoic acid), A-769662, metformin, [(2R,3S,4R,5R)-5-(5-amino-4-carbamoylimidazol-1-yl)-3,4-dihydroxyoxolan-2-yl]methyl dihydrogen phosphate, SB-204990, LY294002, triacscin C , a thiazolidinedione, TCD-717, MN58B, 1,2,3, benzene-tricarboxylate, etomoxir, ranolazine, ST1326, glyburide, perhexiline, cerulenin, C75, TVB-2640, TVB-3166, orlistat, a flavonoid, epigallocatechin-3-gallate, platensimycin, GSK837149A, JZL-184, (6-[4-(2-Bromo-5-methoxy-benzoyl)-piperazin-1-yl]-N-phenylpropyl-nicotinamide), A939572, fatostatin, FGH10019, betulin, and 25-hydroxycholesterol, or any pharmaceutically acceptable salt or prodrugs thereof, or a combination thereof.

14. The inhibitor of lipid metabolism for use according to any one of claims 11 to 13, wherein the cancer is selected from breast cancer, prostate cancer, pancreatic cancer, glioma, glioblastoma, ovarian, endometrial cancer, skin cancer, liver cancer, a Hodgkin lymphoma, a non-Hodgkin lymphoma such as a diffuse large B-cell lymphoma (DLBCL), primary effusion lymphoma, mantle cell lymphoma, Burkitt's lymphoma, follicular lymphoma, Precursor B-cell lymphoblastic leukaemia/lymphoma (B-LBL) or leukemia.
